# FASCICULE DE BREVET EUROPEEN

(11) **EP 4 301 737 B1**
(45) Date de publication et mention de la délivrance du brevet: **23.04.2025**
(21) Numéro de dépôt: 22710664.8
(22) Date de dépôt: 21.02.2022
(51) Int. Cl.: C07D 277/14, C08G 18/10, C08G 18/38, C08G 63/68, C08G 69/26, C08G 69/42

(54) **MONOMÈRES CONTENANT UN MOTIF 4-THIAZOLIDINONE ET LEURS POLYCONDENSATS POLYAMIDE, POLYESTER ET POLYURÉTHANE**
4-THIAZOLIDINONEINHEITEN ENTHALTENDE MONOMERE UND IHRE POLYKONDENSATE POLYAMID, POLYESTER UND POLYURETHAN
MONOMERS CONTAINING A 4-THIAZOLIDINONE UNIT AND THEIR POLYAMIDE, POLYESTER AND POLYURETHANE POLYCONDENSATES

(30) Priorité: 02.03.2021 FR 2102008
(43) Date de publication de la demande: 10.01.2024
(73) Titulaire: Compagnie Générale des Etablissements Michelin, 63000 Clermont-Ferrand (FR)
(72) Inventeur: FEDURCO, Milan, 63040 CLERMONT-FERRAND Cedex 9 (FR); RIBEZZO, Marco, 63040 CLERMONT-FERRAND Cedex 9 (FR)
(74) Mandataire: M.F.P. Michelin
(86) Numéro de dépôt international: PCT/FR2022/050302
(87) Numéro de publication internationale: WO 2022/184994

(56) Documents cités:
- ZHAO YUAN ET AL: "Introducing mercaptoacetic acid locking imine reaction into polymer chemistry as a green click reaction", POLYMER CHEMISTRY, vol. 5, no. 8, 1 January 2014 (2014-01-01), pages 2695 - 2699, XP055805096, ISSN: 1759-9954, DOI: 10.1039/C4PY00058G

## Description

Le domaine de la présente invention est celui des monomères comprenant un motif 4-thiazolidinone destinés à être utilisés dans la synthèse de polyamides, de polyesters et de polyuréthanes obtenus par polycondensation.

La synthèse de polycondensats comprenant des motifs 4-thiazolidinone est décrite dans le journal Polymer Chemistry, 2014, 5, 2695. Elle est réalisée par réaction de polycondensation de l'acide 2-mercaptopropionique, une diamine comme l'hexaméthylènediamine et d'un dialdéhyde comme le téréphtalaldéhyde. Les auteurs de la publication mettent en avant que le procédé de synthèse du polycondensat est respectueux vis-à-vis de l'environnement, car la réaction de polycondensation est conduite à température ambiante en l'absence de catalyseur et génère comme sous-produit seulement de l'eau. Néanmoins, la synthèse du polycondensat s'avère très incommodante pour le voisinage, car elle s'accompagne d'une odeur très désagréable en raison de l'utilisation de l'acide 2-mercaptopropionique lorsque celui-ci est utilisé en grande quantité. Même si les polymères décrits ci-dessus sont stables thermiquement, ils s'avèrent cassants.

Les inventeurs ont mis au point des nouveaux monomères qui permettent de remédier à ces inconvénients dans la synthèse de polycondensats contenant des motifs 4-thiazolidinone, en utilisant des composés, amine ou aldéhyde, qui sont hétérodifonctionnels. Grâce à ces nouveaux monomères, il n'est pas utilisé d'acide 2-mercaptopropionique dans la synthèse de polycondensats contenant des motifs 4-thiazolidinone. En raison de leur structure, les monomères selon l'invention permettent aussi de conduire à une large variété de polycondensats que sont les polyamides, les polyesters et les polyuréthanes et qui ont tous pour point commun de contenir des motifs 4-thiazolidinone dans leurs unités monomères et qui présentent des propriétés thermiques et mécaniques améliorées par rapport au polycondensat contenant des motifs 4-thiazolidinone de l'état de la technique décrit ci-dessus.

Ainsi, un premier objet de l'invention est un monomère qui contient un motif 4-thiazolidinone relié à un noyau benzénique et qui contient deux fonctions choisies parmi hydroxyle et carboxyle, lequel monomère est de formule (I-a) ou (I-b) dans lesquelles Y est hydroxyle ou carboxyle, Q est alkyle ou carboxyalkyle, R₂ est hydrogène, hydroxyle, carboxyle, alkyle ou hydroxyalkyle, Y' est hydroxyle ou carboxyle ; Q' est alkyle ou carboxyalkyle, 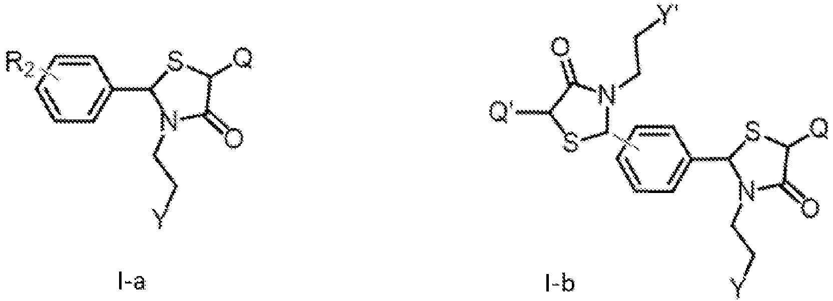 ou de formule (I-c) dans laquelle Y est hydroxyle ou carboxyle, Q est alkyle ou carboxyalkyle, Y' est hydroxyle ou carboxyle ; Q' est alkyle ou carboxyalkyle, x est un entier allant de 0 à 2, 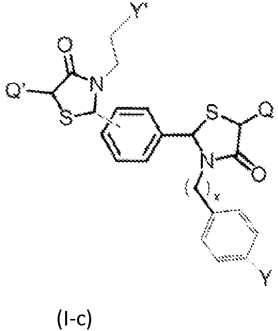 ou bien de formule (II-1) dans laquelle Q est alkyle ou carboxyalkyle, R₃ est hydrogène, hydroxyle, carboxyle, alkyle ou hydroxyalkyle, R₁ est alkyle et A₁, groupe carboné divalent qui contient des atomes de carbone, est un alcanediyle ou un alcanediyle interrompu par un ou plusieurs atomes d'oxygène, m est un entier allant de 0 à 4.

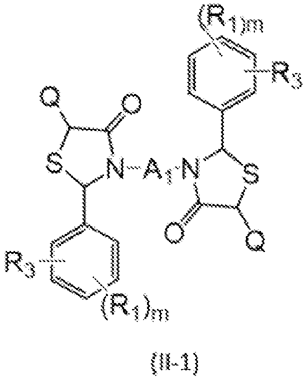

L'invention concerne aussi un procédé de synthèse d'un monomère conforme à l'invention, lequel procédé comprend la réaction d'un aldéhyde de formule (II), d'un composé (III) et d'un composé de formule (IV), le composé (III) étant de formule Y-A-NH₂ ou de formule H₂N-A₁-NH₂ dans lesquelles Y est hydroxyle ou carboxyle, Q est alkyleou carboxyalkyle, A est un éthanediyle, A₁ est un alcanediyle ou un alcanediyle interrompu par un ou plusieurs atomes d'oxygène, R₁ est alkyle, m est un entier allant de 0 à 4, R est hydrogène, hydroxyle, carboxyle, alkyle, hydroxyalkyle ou une fonction aldéhyde.

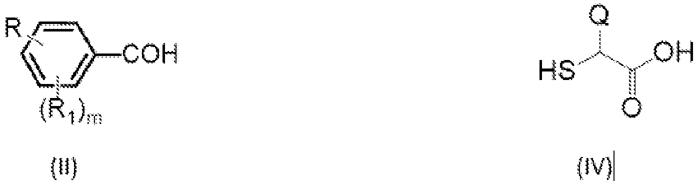

L'invention concerne aussi des procédés de synthèse de polycondensats recourant aux monomères conformes à l'invention.

Ainsi un premier procédé de synthèse de polycondensats conforme à l'invention est un procédé de synthèse d'un polyamide qui comprend la réaction d'un monomère conforme à l'invention avec un deuxième monomère contenant deux fonctions choisies parmi amine et isocyanate, le monomère conforme à l'invention contenant deux fonctions carboxyles.

Un deuxième procédé de synthèse de polycondensats conforme à l'invention est un procédé de synthèse d'un polyester qui comprend la réaction d'un monomère conforme à l'invention avec un deuxième monomère, les deux monomères contenant chacun une fonction carboxyle et une fonction hydroxyle.

Un troisième procédé de synthèse de polycondensats conforme à l'invention et alternatif au deuxième procédé est un procédé de synthèse d'un polyester qui comprend la réaction d'un monomère conforme à l'invention avec un deuxième monomère, l'un des deux monomères contenant deux fonctions hydroxyles et l'autre deux fonctions carboxyles.

Un quatrième procédé de synthèse de polycondensats conforme à l'invention est un procédé de synthèse d'un polyuréthane qui comprend la réaction d'un monomère conforme à l'invention avec un deuxième monomère contenant deux fonctions isocyanates, le monomère conforme à l'invention contenant deux fonctions hydroxyles.

L'invention concerne aussi des procédés de synthèse de polycondensats qui se différencient respectivement des quatre procédés de synthèse de polycondensats que sont le premier, le deuxième, le troisième et le quatrième procédé, par le remplacement du deuxième monomère en partie ou en totalité par un prépolymère qui contient deux fonctions réactives identiques à celle du deuxième monomère.

Un autre objet de l'invention est un polycondensat susceptible d'être obtenu par l'un quelconque des procédés de synthèse de polycondensats conformes à l'invention.

### Description

Les composés mentionnés dans la description peuvent être d'origine fossile ou biosourcés. Dans ce dernier cas, ils peuvent être, partiellement ou totalement, issus de la biomasse ou obtenus à partir de matières premières renouvelables issues de la biomasse. De la même manière, les composés mentionnés peuvent également provenir du recyclage de matériaux déjà utilisés, c'est-à-dire qu'ils peuvent être, partiellement ou totalement, issus d'un procédé de recyclage, ou encore obtenus à partir de matières premières elles-mêmes issues d'un procédé de recyclage.

Dans la présente demande et de manière connue, on entend par hydroxyle le groupe OH, par carboxyle la fonction COOH, par carboxyalkyle un groupe alkyle qui est substitué par la fonction COOH, par hydroxyalkyle un groupe alkyle substitué par la fonction OH.

Dans la présente demande, lorsqu'un symbole représente un atome d'hydrogène, il est dit dans la présente demande qu'il est hydrogène.

On entend par groupe carboné un groupe qui contient des atomes de carbone, notamment un groupe hydrocarboné qui peut être substitué ou interrompu par un ou plusieurs hétéroatomes tels que les atomes d'oxygène, de soufre, d'azote et de silicium.

Lorsque R₂ est différent d'un atome d'hydrogène, R₂ est préférentiellement en para ou en méta par rapport au cycle thiazolidinone, plus préférentiellement en para par rapport au cycle thiazolidinone. Lorsque R₃ est différent d'un atome d'hydrogène, R₃ est préférentiellement en para ou en méta par rapport au cycle thiazolidinone, plus préférentiellement en para par rapport au cycle thiazolidinone. Le nombre de carbone de l'alcanediyle dans A₁ peut varier dans une large mesure. A₁ peut contenir 2 à 20 atomes de carbone. Il est préférentiellement une chaîne linéaire ou ramifiée. Le choix de la structure du groupe carboné divalent A₁ est guidé par les propriétés de flexibilité recherchées pour le polycondensat. Par exemple, la présence d'atomes d'oxygène au sein de la chaîne alcanediyle principale de A₁ pour former des liaisons éther est favorable à l'obtention d'un polycondensat relativement flexible. Un nombre élevé d'atomes de carbone dans une chaîne alcanediyle contribue aussi à augmenter la flexibilité du polycondensat. Préférentiellement A₁ contient au moins 4 atomes de carbone.

Dans la formule (la), x peut prendre les valeurs de 0, de 1 ou de 2 et q est préférentiellement égal à 0.

Les groupes alkyles peuvent être linéaires ou ramifiés. Ils sont préférentiellement linéaires.

Ils contiennent préférentiellement 1 à 12 atomes de carbone.

Les groupes alkyles représentés respectivement par les symboles Q et Q' sont préférentiellement méthyle en raison de la disponibilité commerciale d'un des précurseurs de synthèse du monomère conforme à l'invention, l'acide 2-mercaptopropionique.

Les groupes alkyles représentés par les symboles R₁, R₂, R₃ sont préférentiellement des alkyles ayant 1 à 3 atomes de carbone, plus préférentiellement sont méthyle. Il s'ensuit que le groupe alkyle représenté par le symbole R de la formule (II) du composé utile à la synthèse du monomère est aussi préférentiellement un alkyle ayant 1 à 6 atomes de carbone, plus préférentiellement est méthyle. Les groupes alkyles substitués par un groupe hydroxyle pour constituer le groupe hydroxyalkyle représenté par les symboles R₂ et R₃ sont préférentiellement alkyle ayant 1 à 6 atomes de carbone, plus préférentiellement sont méthyle, auquel cas le groupe hydroxyalkyle est alors de formule CH₂OH. Il s'ensuit que le groupe alkyle substitué par un groupe hydroxyle pour constituer le groupe hydroxyalkyle représenté par le symbole R de la formule (II) du composé utile à la synthèse du monomère est aussi préférentiellement un alkyle ayant 1 à 6 atomes de carbone, plus préférentiellement est méthyle.

Les groupes alkyles substitués par un groupe carboxyle pour constituer les groupes carboxyalkyles représentés respectivement par le symbole Q et Q' sont préférentiellement alkyle ayant 1 à 6 atomes de carbone, plus préférentiellement sont méthyle, auquel cas le groupe carboxyalkyle est alors de formule CH₂COOH.

De manière préférentielle, Q et Q' sont méthyle ou carboxyméthyle.

De préférence, R₂ est hydrogène, hydroxyle, carboxyle ou hydroxyméthyle.

R₃ est de préférence hydroxyle ou carboxyle. Lorsque R₃ est hydroxyle ou carboxyle, Q est préférentiellement alkyle, plus préférentiellement méthyle.

Dans la formule (II-1), m est préférentiellement égal à 0.

Selon une première variante de l'invention, le monomère est de formule (I-a) ou (I-b) dans lesquelles Y est hydroxyle ou carboxyle, Q est alkyle ou carboxyalkyle, R₂ est hydrogène, hydroxyle, carboxyle, alkyle ou hydroxyalkyle, Y' est hydroxyle ou carboxyle ; Q' est alkyle ou carboxyalkyle. De préférence dans les formules (I-a) et (I-b), R₂ est hydrogène, hydroxyle, carboxyle ou hydroxyméthyle et Q et Q' sont méthyle ou carboxyméthyle.

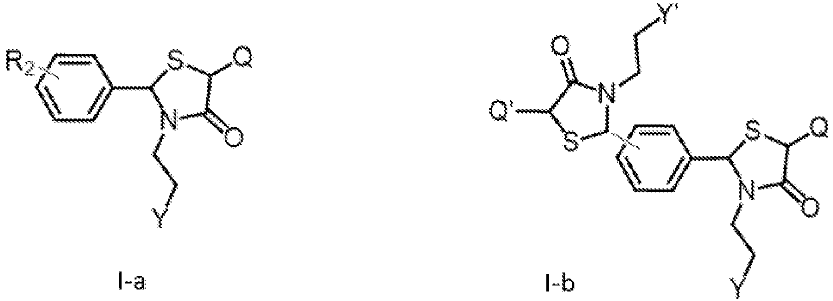

Des monomères selon la première variante sont tout particulièrement les composés (1) à (8) suivants :

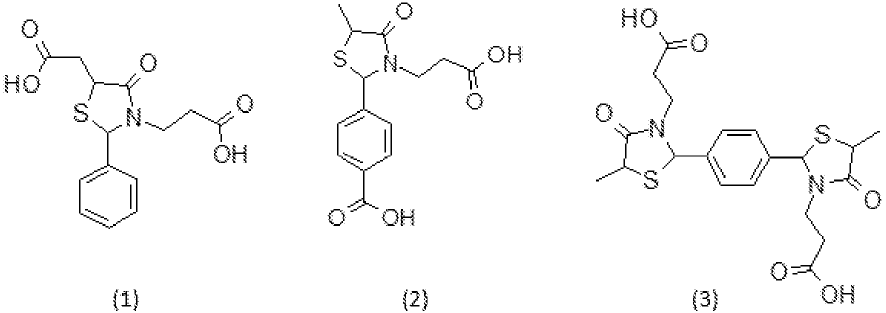

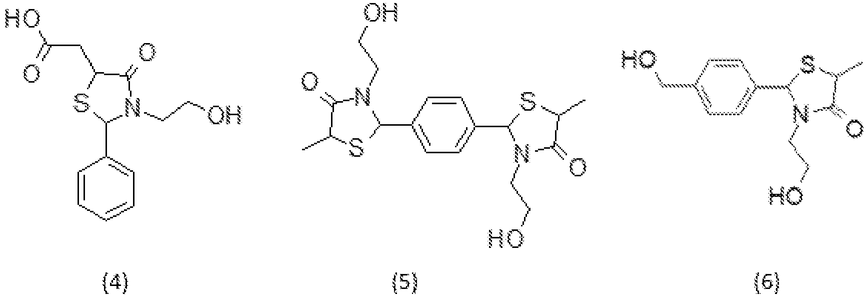

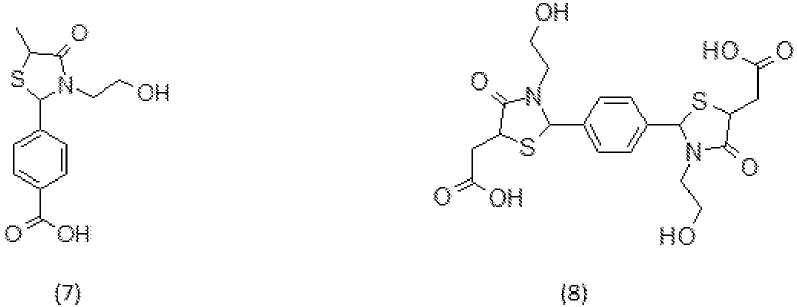

Selon un premier mode de réalisation de la première variante, le monomère est de formule (I-a) dans laquelle R₂ est hydrogène, Y est hydroxyle ou carboxyle et Q est carboxyalkyle, préférentiellement carboxyméthyle. Les monomères qui satisfont à ce premier mode sont préférentiellement les composés (4) et (1).

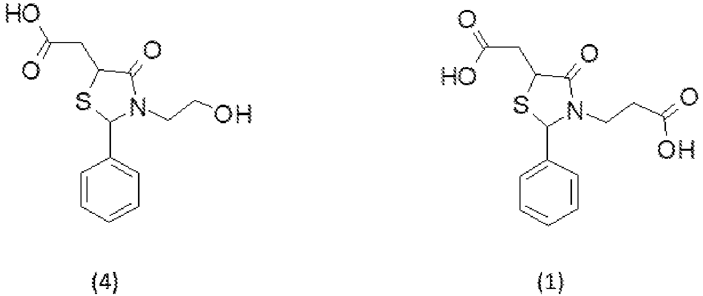

Selon un deuxième mode de réalisation de la première variante, le monomère est de formule (I-a) dans laquelle R₂ est carboxyle, Y est hydroxyle ou carboxyle, Q est alkyle, en particulier méthyle. Les monomères qui satisfont à ce deuxième mode sont préférentiellement les composés (7) et (2).

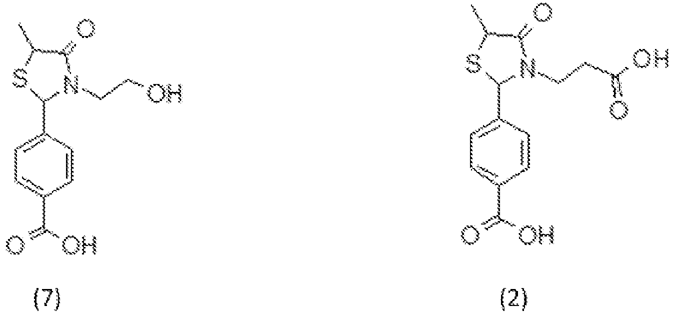

Selon un troisième mode de réalisation de la première variante, le monomère est de formule (I-a) dans laquelle R₂ est hydroxyalkyle, préférentiellement hydroxyméthyle, Y est hydroxyle ou carboxyle, Q est alkyle, en particulier méthyle. Un monomère qui satisfait à ce troisième mode est préférentiellement le composé (6).

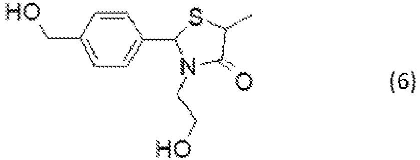

Selon un quatrième mode de réalisation de la première variante, le monomère est de formule (I-b) dans laquelle Y et Y'sont hydroxyle ou carboxyle, Q et Q' sont alkyle ou carboxyalkyle, en particulier méthyle ou carboxyméthyle. Les monomères qui satisfont à ce quatrième mode sont préférentiellement les composés (3) et (5).

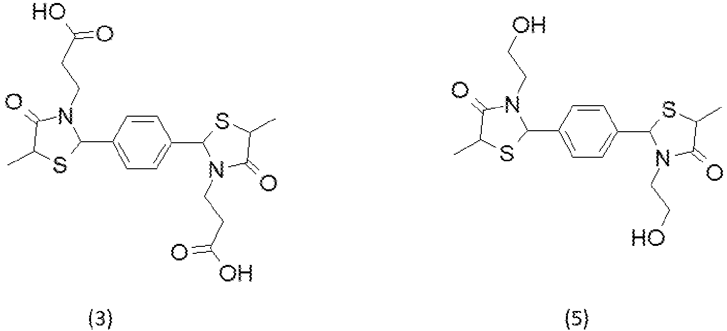

Selon un cinquième mode de réalisation de la première variante, le monomère est de formule (I-b) dans laquelle Y et Y'sont hydroxyle ou carboxyle, Q et Q' sont carboxyalkyle, en particulier carboxyméthyle. Un monomère qui satisfait à ce cinquième mode est préférentiellement le composé (8).

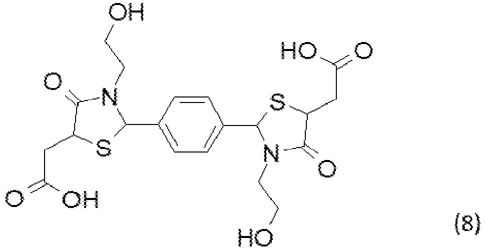

Selon une deuxième variante de l'invention, le monomère est de formule (I-c) dans lesquelles Y est hydroxyle ou carboxyle, Q est alkyle ou carboxyalkyle, Y' est hydroxyle ou carboxyle ; Q' est alkyle ou carboxyalkyle, x est un entier allant de 0 à 2.

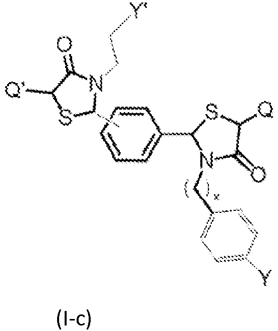

Dans la formule (I-c), x peut être égal à 0, à 1 ou à 2. Dans la formule (I-c), Q et Q' sont préférentiellement méthyle. Un monomère selon la deuxième variante est tout particulièrement le composé (12).

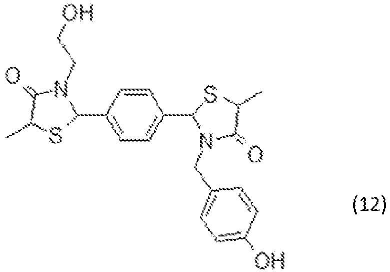

Selon une troisième variante de l'invention, le monomère est de formule (II-1) dans laquelle Q est alkyle ou carboxyalkyle, R₃ est hydrogène, hydroxyle, carboxyle, alkyle ou hydroxyalkyle et A₁ est un alcanediyle ou un alcanediyle interrompu par un ou plusieurs atomes d'oxygène.

Comme monomères satisfaisant à la formule (II-1), on peut mentionner les composés (13) à (18).

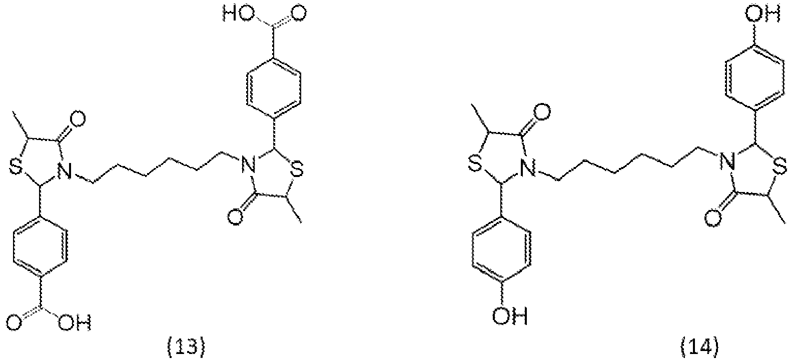

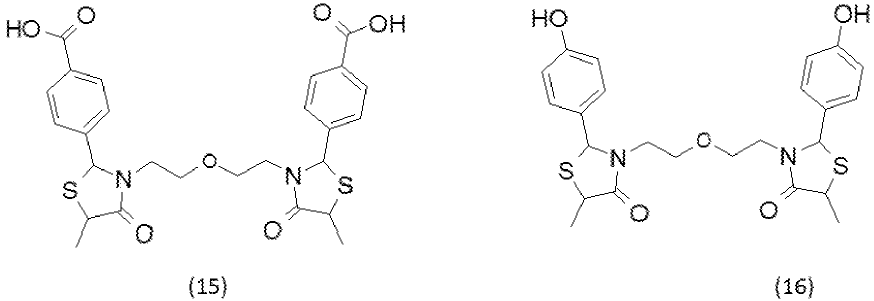

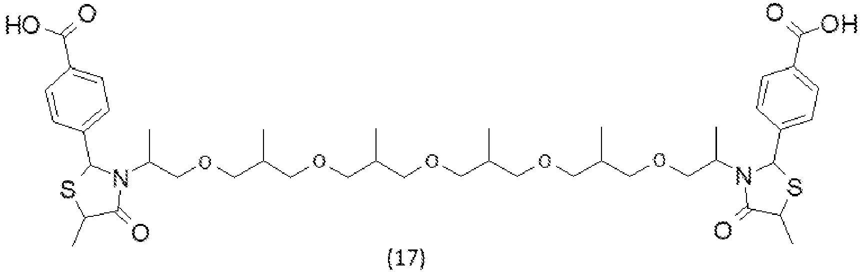

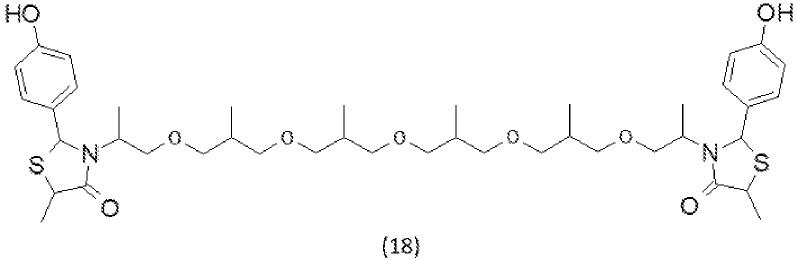

Le monomère conforme à l'invention est préparé typiquement par un procédé, autre objet de l'invention, qui comprend la réaction d'un aldéhyde de formule (II), d'un composé (III) et d'un composé de formule (IV) 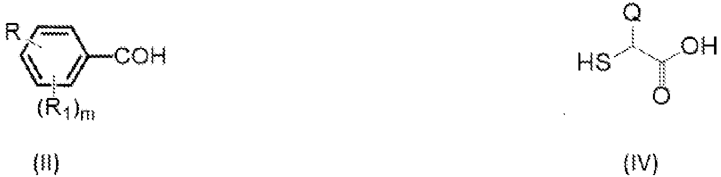
le composé (III) étant de formule Y-A-NH₂ ou de formule H₂N-A₁-NH₂,
dans lesquelles Y est hydroxyle ou carboxyle, Q est alkyle ou carboxyalkyle, A est un éthanediyle, A₁ est un alcanediyle ou un alcanediyle interrompu par un ou plusieurs atomes d'oxygène, R₁ est alkyle, m est un entier allant de 0 à 4, R est hydrogène, hydroxyle, carboxyle, alkyle, hydroxyalkyle, ou une fonction aldéhyde. Un des symboles Y, Q et R contient une fonction hydroxyle (OH) ou carboxyle (COOH). Lorsque R représente une fonction aldéhyde, le procédé conduit généralement à un monomère de formule (I-b). Le groupe carboné A₁ du composé (III) est de même structure chimique que son homologue dans les monomères conformes à l'invention.

Les monomères peuvent être préparés selon les schémas réactionnels 1 à 4.

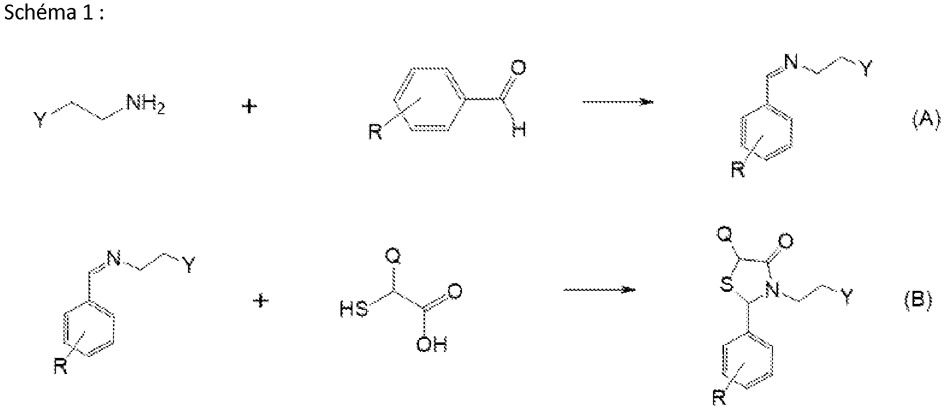

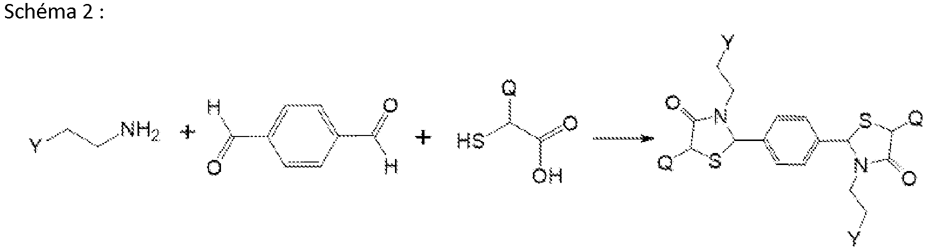

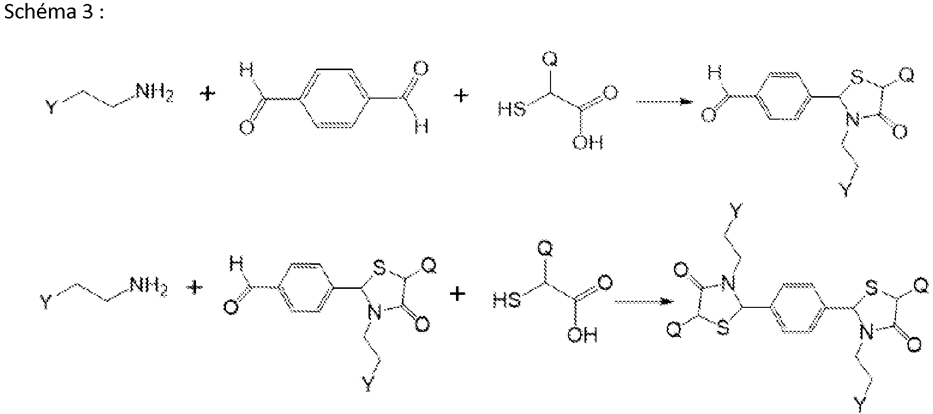

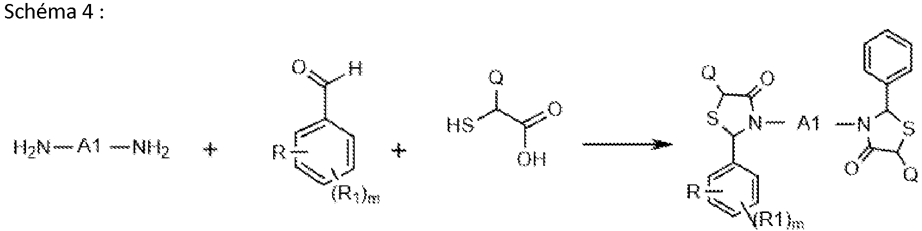

Par exemple, des monomères selon le premier mode de réalisation de la première variante peuvent être préparés par un procédé mettant en jeu l'une ou l'autre des réactions suivantes :

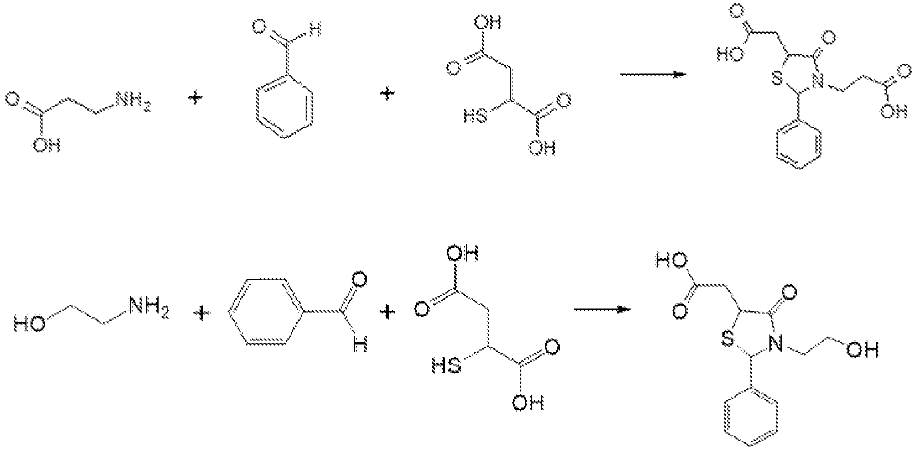

Par exemple, des monomères selon le deuxième mode de réalisation de la première variante peuvent être préparés par un procédé mettant en jeu l'une ou l'autre des réactions suivantes :

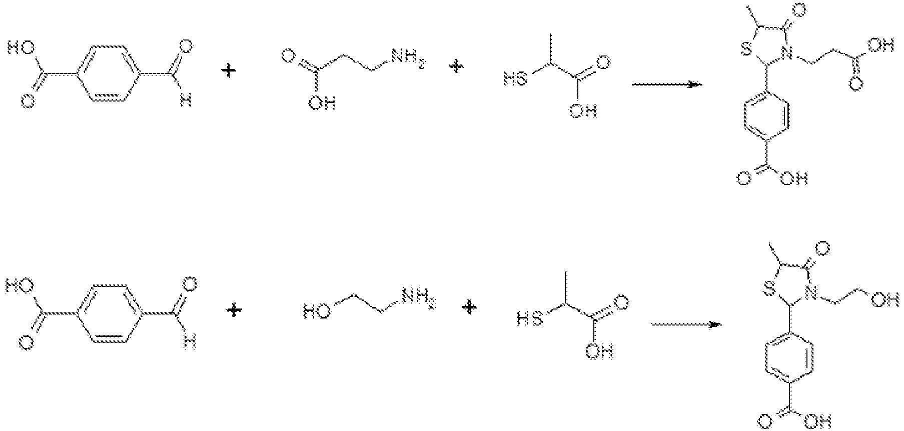

Par exemple, des monomères selon le troisième mode de réalisation de la première variante peuvent être préparés par un procédé mettant en jeu la réaction suivante :

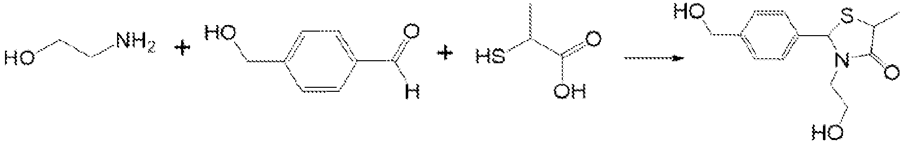

Par exemple, des monomères selon le quatrième mode de réalisation de la première variante peuvent être préparés par un procédé mettant en jeu l'une ou l'autre des réactions suivantes :

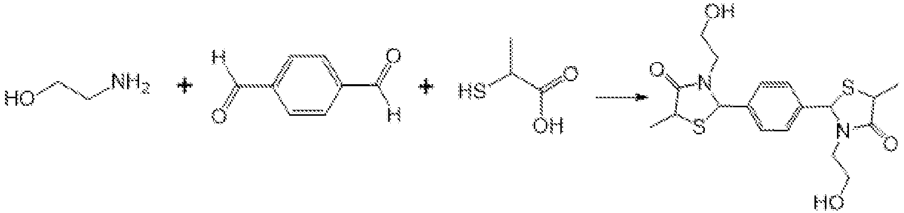

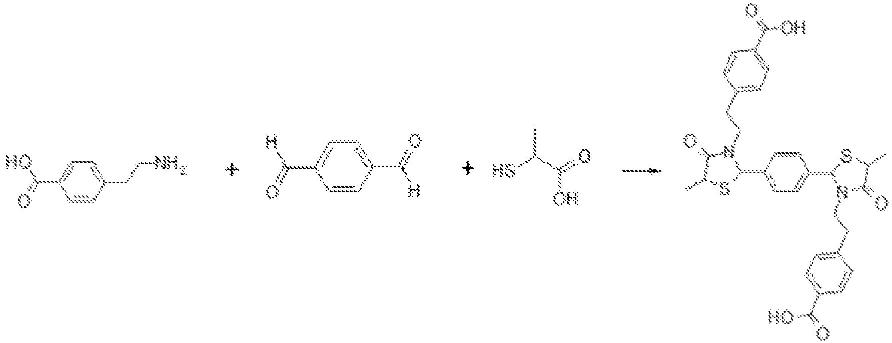

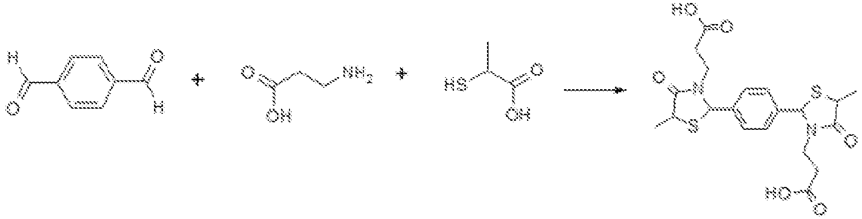

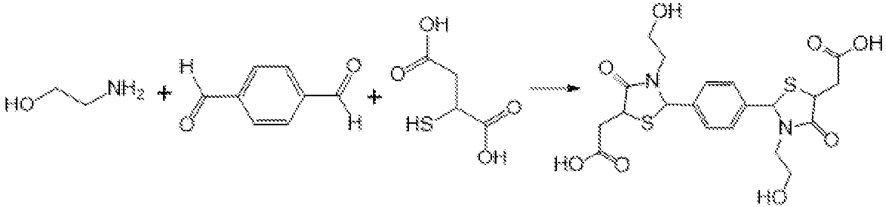

Par exemple, des monomères selon la deuxième variante peuvent être préparés par un procédé mettant en jeu le schéma réactionnel suivant :

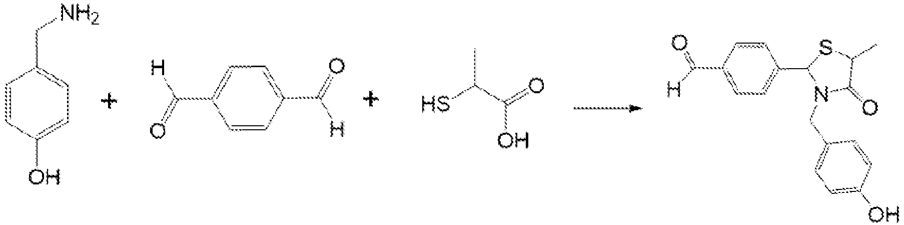

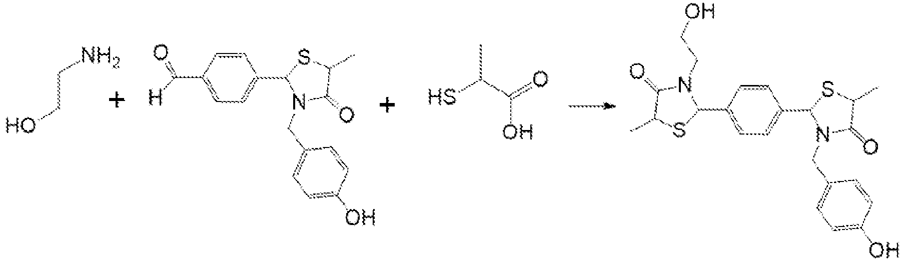

Par exemple des monomères selon la troisième variante peuvent être préparés par un procédé mettant en jeu le schéma réactionnel suivant :

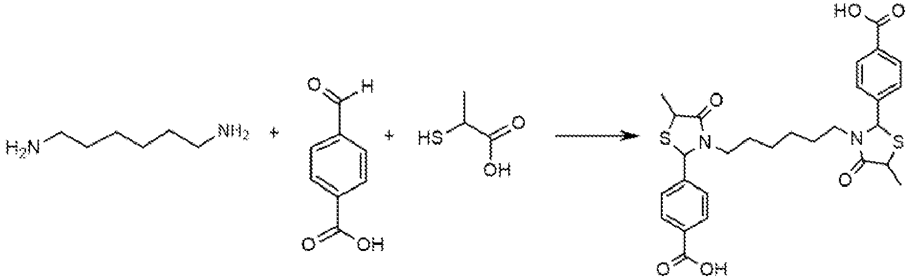

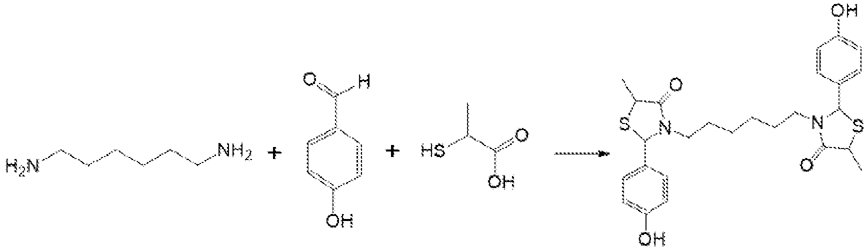

Les conditions réactionnelles pour synthétiser le monomère sont adaptées par l'homme du métier selon le schéma réactionnel mis en jeu et en tenant compte de la solubilité de chacun des substrats et de la stœchiométrie de la réaction.

Les monomères conformes à l'invention sont utilisés dans des procédés de synthèse de polycondensats, respectivement de polyamide, de polyester et de polyuréthane. Dans les procédés de synthèse des polycondensats, les deux fonctions réactives du monomère conforme à l'invention, qui sont choisies parmi hydroxyle et carboxyle, peuvent se présenter respectivement sous la forme d'un hydroxylate (O⁻) ou d'un carboxylate (COO⁻), auquel cas le monomère se présente sous la forme d'un sel dans le procédé de synthèse du polycondensat. Les deux fonctions réactives du monomère conforme à l'invention sont celles qui participent à la réaction de polycondensation avec un autre monomère dit deuxième monomère pour former un polycondensat. Le deuxième monomère est typiquement un composé qui contient deux fonctions qui sont réactives respectivement avec les deux fonctions réactives du monomère conforme à l'invention. Les deux fonctions du deuxième monomère sont reliées l'une à l'autre de façon covalente par l'intermédiaire d'un espaceur, chaîne hydrocarbonée divalente, aromatique ou aliphatique, éventuellement interrompue par un ou plusieurs hétéroatomes comme l'oxygène, l'azote ou le soufre. Le nombre de carbone de l'espaceur peut varier dans une large mesure. Il varie préférentiellement de 4 à 20. Comme chaîne hydrocarbonée divalente, on peut citer les alcanediyles, les arènediyles, les alkylarènediyles. Le deuxième monomère peut être aussi un monomère selon l'invention et être de formule (I-a), (I-b), (I-c) ou (II-1).

Lorsque le monomère conforme à l'invention a pour fonction réactive deux fonctions carboxyles, il réagit selon un premier procédé de synthèse de polycondensats avec un deuxième monomère qui contient deux fonctions choisies parmi amine et isocyanate pour former un polyamide. Lorsque le monomère est de formule (I-a), deux des symboles Y, Q et R₂ sont carboxyles ; lorsque le monomère est de formule (I-b), deux des symboles Y, Q, Y' et Q' sont carboxyles ; lorsque le monomère est de formule (II-1), deux des symboles R₃ et Q sont carboxyles. Dans le premier procédé peuvent être utilisés les composés de formule (1), (2), (3), (8), (13), (15) et (17). Le deuxième monomère peut être une diamine ou un diisocyanate. De façon équivalente, les deux monomères réagissent sous la forme de leur sel, le monomère conforme à l'invention étant sous la forme d'un sel de carboxylate, le deuxième monomère étant une diamine sous la forme d'un sel d'hydrohalogénate.

Comme deuxième monomère conviennent par exemple l'hexaméthylènediamine ou 1,6 diaminohexane, le 1,12-diaminodocédane, le diphénylmethane diisocyanate ("MDI"), le toluène diisocyanate ("TDI"), le naphthalène diisocyanate ("NDI"), le 3,3'-bitoluène diisocyanate ("TODI"), le para-phénylene diisocyanate ("PPDI"), le 1,4-tétraméthylène diisocyanate, le 1,6-hexane diisocyanate ("HDI"), le 1,4-bis(isocyanatoméhyl)cyclohexane, le 1,3-bis(isocyanatométhyl)cyclohexane, 1,3-bis(isocyanatométhyl)benzène, le 1,4-bis(isocyanatométhyl)benzène, l'isophorone diisocyanate ("IPDI"), le bis(4-isocyanatocyclohexyl) méthane diisocyanate ("H12MDI") et le 4,4'-dicyclohexylméthane diisocyanate ("H13MDI").

Lorsque le monomère conforme à l'invention a pour fonction réactive une fonction carboxyle et une fonction hydroxyle, il réagit selon un deuxième procédé de synthèse de polycondensats avec un deuxième monomère qui contient lui aussi une fonction carboxyle et une fonction hydroxyle pour former un polyester. Lorsque le monomère est de formule (I-a), deux des symboles Y, Q et R₂ sont respectivement carboxyle et hydroxyle ; lorsque le monomère est de formule (I-b), deux des symboles Y, Q, Y' et Q' sont respectivement carboxyle et hydroxyle. Dans le premier procédé peuvent être utilisés les composés de formule (4), (7) et (8). Lorsque le monomère est de formule (II-1), deux des symboles Q et R₃ sont respectivement carboxyle et hydroxyle.

Lorsque le monomère conforme à l'invention tel que les composés de formule (5), (6), (8), (14), (16) et (18) a pour fonction réactive deux fonctions hydroxyles, il réagit selon un troisième procédé de synthèse de polycondensats avec un deuxième monomère qui contient deux fonctions carboxyles pour former un polyester. Comme deuxième monomère convient tout diacide, aliphatique ou aromatique, tel que l'acide 1,4-butanedioïque, l'acide téréphtalique, l'acide isophtalique ou encore les composés de formule (1), (2), (3), (8), (13), (15) et (17).

Alternativement pour former un polyester selon le troisième procédé, le monomère conforme à l'invention tel que les composés de formule (1), (2), (3), (8), (13), (15) et (17) a pour fonction réactive deux fonctions carboxyles, le deuxième monomère contient deux fonctions hydroxyles. Comme deuxième monomère convient tout diol, aliphatique ou aromatique, tel que le 1,4-butanediol, l'hydroquinone bis(2-hydroxyéthyléther) (HQEE), le résorcinol bis(2-hydroxyéthyléther) (HER) ou bien les composés de formule (5), (6) et (8).

Lorsque le monomère conforme à l'invention tel que les composés de formule (5), (6), (8), (14), (16) et (18) a pour fonction réactive deux fonctions hydroxyles, il réagit selon un quatrième procédé de synthèse de polycondensats avec un deuxième monomère qui contient deux fonctions isocyanates pour former un polyuréthane. Le deuxième monomère est typiquement un diisocyanate tel que défini dans le premier procédé de synthèse de polycondensats.

Dans les procédés de synthèse de polycondensats conformes à l'invention, le deuxième monomère peut être remplacé en partie ou en totalité par un prépolymère qui contient deux fonctions réactives identiques à celle du deuxième monomère. Le terme prépolymère est utilisé selon le sens donné par IUPAC dans le Gold Book. Comme prépolymère conviennent par exemples les oligomères suivants, p étant égal ou supérieur à 1 :

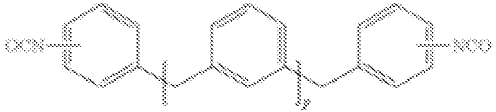

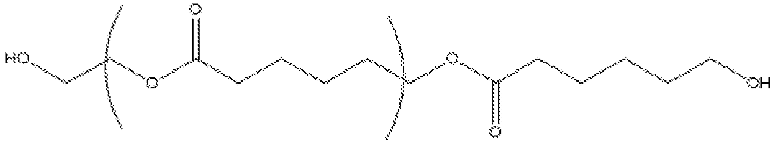

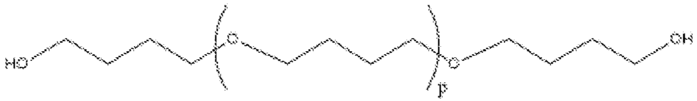

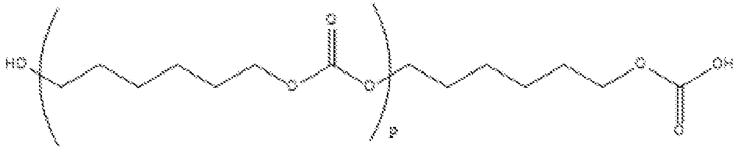

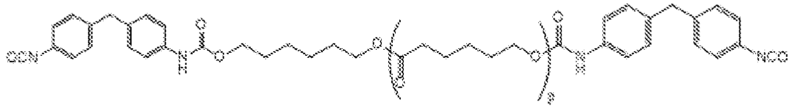

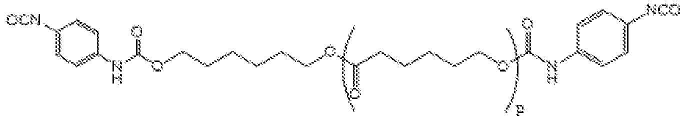

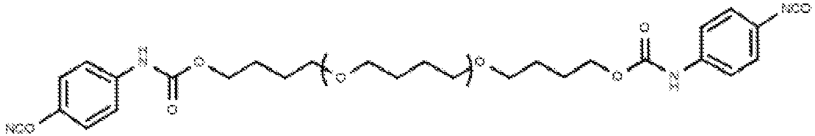

Les 3 prépolymères isocyanates sont appelés prépolymère MDI pour le premier et PPDI pour les deux autres.

Les conditions réactionnelles pour former les polycondensats selon les procédés conformes à l'invention sont celles communément mises en œuvre dans la synthèse de polyamides et sont bien connues de l'homme du métier. L'homme du métier les adapte en fonction de la solubilité des réactifs et de la stœchiométrie de la réaction.

Les polycondensats, susceptibles d'être obtenus par les procédés de synthèse de polycondensats conformes à l'invention, sont des polycondensats contenant des unités monomères comportant des motifs 4-thiazolidinone. Ainsi, un polycondensat qui peut être obtenu par le premier procédé de synthèse de polycondensat selon l'invention est un polyamide qui contient des unités monomères comportant des motifs 4-thiazolidinone ; un polycondensat qui peut être obtenu par le deuxième ou le troisième procédé de synthèse de polycondensat selon l'invention est un polyester qui contient des unités monomères comportant des motifs 4-thiazolidinone ; un polycondensat qui peut être obtenu par le quatrième procédé de synthèse de polycondensat selon l'invention est un polyuréthane qui contient des unités monomères comportant des motifs 4-thiazolidinone.

Les polycondensats polyamide, polyester et polyuréthane selon l'invention présentent des propriétés thermiques améliorées par rapport à leurs homologues respectifs polyamide, polyester et polyuréthane qui ne contiennent pas de motifs 4-thiazolidinone.

En résumé, l'invention est mise en œuvre avantageusement selon l'un quelconque des modes de réalisation suivants 1 à 24 :
Mode 1 : Monomère qui contient un motif 4-thiazolidinone relié à un noyau benzénique et qui contient deux fonctions choisies parmi hydroxyle et carboxyle, lequel monomère est de formule de formule (I-a) ou (I-b) dans lesquelles Y est hydroxyle ou carboxyle, Q est alkyle ou carboxyalkyle, R₂ est hydrogène, hydroxyle, carboxyle, alkyle ou hydroxyalkyle, Y' est hydroxyle ou carboxyle ; Q' est alkyle ou carboxyalkyle, 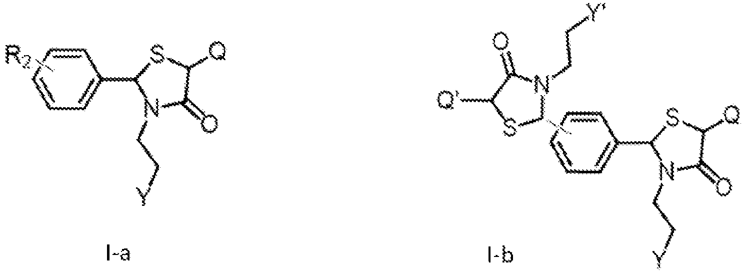 ou de formule (I-c) dans laquelle Y est hydroxyle ou carboxyle, Q est alkyle ou carboxyalkyle, Y' est hydroxyle ou carboxyle ; Q' est alkyle ou carboxyalkyle, x est un entier allant de 0 à 2, 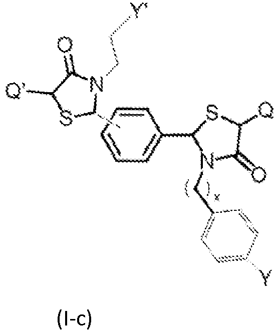 ou bien de formule (II-1) dans laquelle Q est alkyle ou carboxyalkyle, R₁ est alkyle, R₃ est hydrogène, hydroxyle, carboxyle, alkyle ou hydroxyalkyle et A₁, groupe carboné divalent qui contient des atomes de carbone, est un alcanediyle ou un alcanediyle interrompu par un ou plusieurs atomes d'oxygène, m est un entier allant de 0 à 4. 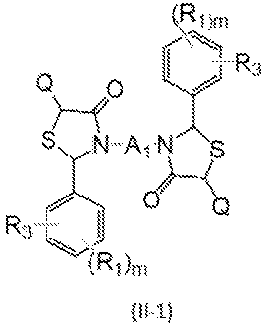
Mode 2 : Monomère selon le mode 1 dans lequel R₂ et R₃ sont en para ou en méta par rapport au cycle thiazolidinone lorsqu'ils sont différents d'un atome d'hydrogène.
Mode 3 : Monomère selon le mode 1 ou 2 dans lequel R₂ et R₃ sont en para par rapport au cycle thiazolidinone lorsqu'ils sont différents d'un atome d'hydrogène.
Mode 4 : Monomère selon l'un quelconque des modes 1 à 3 dans lequel m est égal à 0.
Mode 5 : Monomère selon l'un quelconque des modes 1 à 4 dans lequel les alkyles représentés par Q et Q' sont méthyle.
Mode 6 : Monomère selon l'un quelconque des modes 1 à 5 dans lequel les carboxyalkyles représentés par Q et Q' sont carboxyméthyle.
Mode 7 : Monomère selon l'un quelconque des modes 1 à 6 dans lequel les hydroxyalkyles représentés par R₂ et R₃ sont hydroxyméthyle.
Mode 8 : Monomère selon l'un quelconque des modes 1 à 7 dans lequel les alkyles représentés par R₁, R₂, R₃ sont des alkyles ayant 1 à 3 atomes de carbone.
Mode 9 : Monomère selon l'un quelconque des modes 1 à 8 dans lequel les alkyles représentés par R₁, R₂, R₃ sont méthyle.
Mode 10 : Monomère selon l'un quelconque des modes 1 à 9 dans lequel Q et Q' sont alkyle ou carboxyalkyle.
Mode 11 : Monomère selon l'un quelconque des modes 1 à 10 dans lequel A₁ contient 2 à 20 atomes de carbone.
Mode 12 : Monomère selon l'un quelconque des modes 1 à 11 dans lequel A₁ contient au moins 4 atomes de carbone.
Mode 13 : Monomère selon l'un quelconque des modes 1 à 12, lequel monomère est de formule (I-a) ou (I-b) dans lesquelles Y est hydroxyle ou carboxyle, Q est alkyle ou carboxyalkyle, R₂ est hydrogène, hydroxyle, carboxyle, alkyle ou hydroxyalkyle, Y' est hydroxyle ou carboxyle ; Q' est alkyle ou carboxyalkyle. 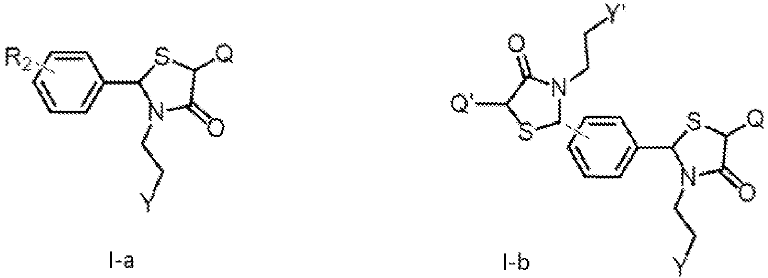
Mode 14 : Monomère selon l'un quelconque des modes 1 à 10, lequel monomère est de formule (I-c) dans laquelle Y est hydroxyle ou carboxyle, Q est alkyle ou carboxyalkyle, Y' est hydroxyle ou carboxyle ; Q' est alkyle ou carboxyalkyle, x est un entier allant de 0 à 2. 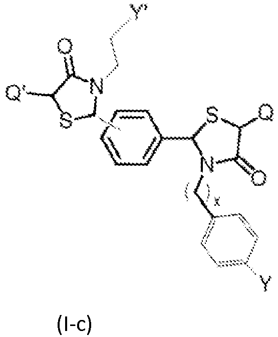
Mode 15 : Monomère selon l'un quelconque des modes 1 à 14 dans lequel R₂ est hydrogène, hydroxyle, carboxyle, hydroxyalkyle.
Mode 16 : Monomère selon l'un quelconque des modes 1 à 11, lequel monomère est de formule (II-1) dans laquelle Q est alkyle ou carboxyalkyle, R₃ est hydrogène, hydroxyle, carboxyle, alkyle ou hydroxyalkyle et A₁ est un alcanediyle ou un alcanediyle interrompu par un ou plusieurs atomes d'oxygène.
Mode 17 : Monomère selon l'un quelconque des modes 1 à 11 ou 16 dans lequel R₃ est hydroxyle ou carboxyle.
Mode 18 : Procédé de synthèse d'un monomère défini à l'un quelconque des modes 1 à 17 qui comprend la réaction d'un aldéhyde de formule (II), d'un composé (III) et d'un composé de formule 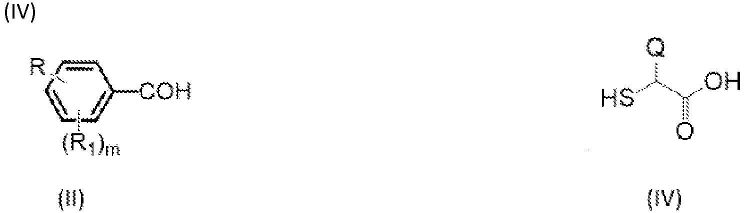
   le composé (III) étant de formule Y-A-NH₂ ou de formule H₂N-A₁-NH₂,
   dans lesquelles Y est hydroxyle ou carboxyle, Q est hydrogène, alkyle ou carboxyalkyle, A est éthanediyle, A1 est un alcanediyle ou un alcanediyle interrompu par un ou plusieurs atomes d'oxygène, R₁ est alkyle, m est un entier allant de 0 à 4, R est hydrogène, hydroxyle, carboxyle, alkyle, hydroxyalkyle, ou une fonction aldéhyde.
Mode 19 : Procédé de synthèse d'un polyamide qui comprend la réaction d'un monomère défini à l'un quelconque des modes 1 à 17 avec un deuxième monomère contenant deux fonctions choisies parmi amine et isocyanate, le monomère de formule défini à l'un quelconque des modes 1 à 17 contenant deux fonctions carboxyles.
Mode 20 : Procédé de synthèse d'un polyester qui comprend la réaction d'un monomère défini à l'un quelconque des modes 1 à 17 avec un deuxième monomère, les deux monomères contenant chacun une fonction carboxyle et une fonction hydroxyle ou bien l'un des deux monomères contenant deux fonctions hydroxyles et l'autre deux fonctions carboxyles.
Mode 21 : Procédé de synthèse d'un polyester qui comprend la réaction d'un monomère défini à l'un quelconque des modes 1 à 17 avec un deuxième monomère, l'un des deux monomères contenant deux fonctions hydroxyles et l'autre deux fonctions carboxyles.
Mode 22 : Procédé de synthèse d'un polyuréthane qui comprend la réaction d'un monomère défini à l'un quelconque des modes 1 à 17 avec un deuxième monomère contenant deux fonctions isocyanates, le monomère de formule défini à l'un quelconque des modes 1 à 17 contenant deux fonctions hydroxyles.
Mode 23 : Procédé selon l'un quelconque des modes 19 à 22 dans lequel le deuxième monomère est remplacé en partie ou en totalité par un prépolymère qui contient deux fonctions réactives identiques à celle du deuxième monomère.
Mode 24 : Polycondensat susceptible d'être obtenu par un procédé défini à l'un quelconque des modes 19 à 22, le polycondensat étant un polyamide, un polyester ou un polyuréthane et contenant des unités monomères comportant des motifs 4-thiazolidinone.

Les caractéristiques précitées de la présente invention, ainsi que d'autres, seront mieux comprises à la lecture de la description suivante de plusieurs exemples de réalisation de l'invention, donnés à titre illustratif et non limitatif.

### Exemples

Les caractérisations thermiques par calorimétrie différentiel à balayage (DSC) ont été réalisées avec un appareil Mettler Toledo DSC3. L'échantillon (10 mg environ) est pesé et scellé dans un creuset en aluminium de 40 µL. Le creuset est percé avec une fine aiguille juste avant la mesure. De l'azote sec est utilisé comme gaz de purge avec un flux de 30 mL min⁻¹. Deux cycles successifs de chauffage et de refroidissement sont réalisés à l'identique. Pour chaque cycle, l'échantillon ainsi qu'un creuset vide de référence sont chauffés depuis une température minimale à une température maximale à 10°C min⁻¹. La température est maintenue 2 minutes à la température maximale, puis il est refroidi de la température maximale à la température minimale à 10°C min⁻¹. Sauf indication contraire, la température de transition vitreuse (Tg) et la température de fusion (Tf) sont mesurées au second cycle. Le cas échéant, en cas de perte de masse observée au cours du deuxième cycle, un troisième cycle est réalisé dans les mêmes conditions que le deuxième cycle. La température de transition vitreuse (Tg) correspond à la température T_{mg} « midpoint temperature » telle que définie dans la norme ASTM D3418-99. La température de fusion (Tf) correspond à la pointe du pic de fusion.

L'analyse mécanique dynamique (DMA) en traction/compression a été réalisée sur une éprouvette de 24 mm de longueur, de 3 mm d'épaisseur et de 5 mm de largeur avec un appareil Metravib DMA40+ à une fréquence de 10 Hz avec un balayage en température de -120°C à 180°C avec une rampe de 2°C/min, sauf indication contraire.

Par analyse DMA sont mesurés la température de transition vitreuse et le module de Young (E') à une température donnée. La température de transition vitreuse mesurée par DMA correspond à la température à laquelle le module de perte (E") atteint son maximum.

Toutes les synthèses sont conduites sous atmosphère d'azote, sauf indications contraires.

### Monomère selon l'invention, composé (6) : Exemple 1

### Synthèse du 3-(2-hydroxy-éthyl)-2-(4-hydroxyméthyl-phényl)-5-méthyl-thiazolidin-4-one par réaction de l'acide 2-mercaptopropionique, du 2-aminoéthanol et du 4-(hydroxyméthyl) benzaldéhyde :

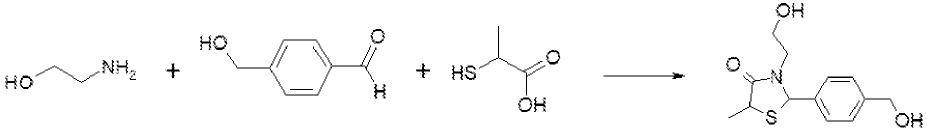

Dans un ballon à 4 cols de 100 ml préalablement séché sous vide à plus de 100°C, équipé d'un condenseur, bulleur, thermomètre et barreau magnétique, sont introduits 4.085 g (soit 30 mmol) de 4-(hydroxyméthyl) benzaldéhyde (99%, Fluorochem, #222581) suivi de 1.83 g (soit 30 mmol) d'éthanolamine. On observe une exothermie à environ 40°C et tout devient rapidement soluble sous agitation. On ajoute pour terminer 3.35 g (soit 30 mmol) de l'acide 2-mercaptopropionique à 95%. Le milieu réactionnel est chauffé à 75°C et agité durant 18 heures sous léger débit d'azote. Le monomère est purifié par chromatographie avec le mélange acétate d'éthyle et l'heptane (4 : 1), suivi d'acétate d'éthyle et méthanol (95 :5). La structure du produit est confirmée par analyse de résonance magnétique nucléaire (RMN).

¹H RMN (600 MHz, DMSO-d6) δ 7.54 - 7.11 (m, 4H), 5.81 (s, 1H), 5.20 (t, J = 5.7 Hz, 1H), 4.74 (s, 1H), 4.48 (t, J = 5.0 Hz, 2H), 3.99 (dd, J = 27.0, 7.1 Hz, 1H), 3.53 (d, J = 13.8 Hz, 1H), 3.40 (s, 1H), 3.29 (s, 1H), 2.59 (d, J = 13.7 Hz, 1H), 1.45 (dd, J = 25.5, 7.0 Hz, 3H).

### Monomère selon l'invention, composé (4) : Exemple 2

### Synthèse de l'acide [3-(2-hydroxy-éthyl)-4-oxo-2-phényl -thiazolidin-5-yl]-acétique par réaction de l'acide mercaptosuccinique, d'éthanolamine et du benzaldéhyde :

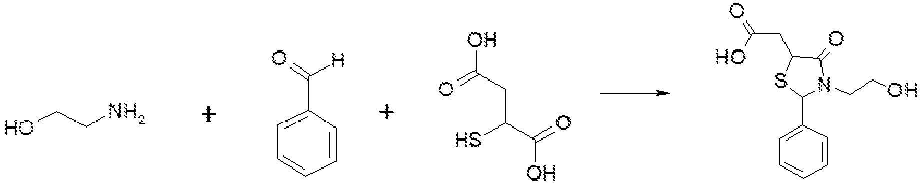

Dans un ballon à 4 cols de 100 ml préalablement séché sous vide à plus de 100°C, équipé d'un condenseur, bulleur, thermomètre et barreau magnétique, sont introduits 6.96 g (soit 65.6 mmol) de benzaldéhyde suivi de 10 ml de méthanol anhydre. Sous agitation, 4.0 g (soit 65.6 mmol) d'éthanolamine sont ajoutés et on constate une exothermie à environ 40°C. Le mélange réactionnel est refroidi à température ambiante à l'aide d'un bain de glace et on ajoute pour terminer 9.85 g (soit 65.6 mmol) d'acide mercaptosuccinique (99%, Alrich #88460). Le milieu réactionnel est agité à température ambiante durant 65 heures sous léger débit d'azote. Le méthanol est évaporé et on obtient un liquide visqueux (miel) jaune claire. Après purification de 9.8g de produit brut sur gel de silice (éluant pentane/acétate d'éthyle (70/30 en volume), puis acétate d'éthyle), on isole 5.2g de solide blanc.

¹H RMN (600 MHz, DMSO-d6) δ 12.54 (s, 1H), 7.57 - 7.20 (m, 5H), 5.84 (s, 1H), 4.75 (t, *J* = 5.6 Hz, 1H), 4.38 - 4.08 (m, 1H), 3.55 (dt, *J* = 13.7, 6.0 Hz, 1H), 3.42 (dd, *J* = 11.2, 5.8 Hz, 1H), 3.10 (dd, *J* = 17.1, 3.6 Hz, 1H), 2.67 (dd, *J* = 17.1, 10.1 Hz, 1H), 2.58 (dt, *J* = 13.7, 6.2 Hz, 1H).

### Monomère selon l'invention, composé (5) : Exemple 3

### Synthèse du 2,2'-(1,4-phénylène) bis[3-(2-hydroxyéthyl)-5-méthyl-1,3-thiazolidin-4-one] par réaction du téréphtalaldéhyde, d'éthanolamine et d'acide 2-mercaptopropionique :

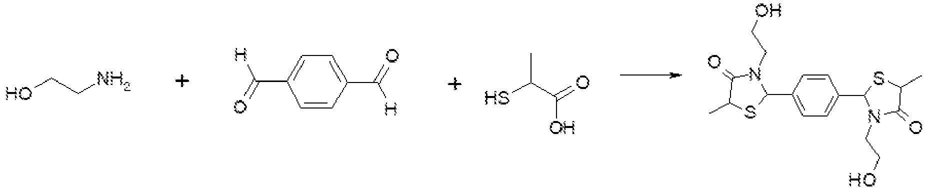

Dans un ballon à quatre cols de 100 ml préalablement séché sous vide à plus de 100°C, équipé d'un condenseur, bulleur, thermomètre et barreau magnétique, sont introduits 13.4 g (soit 100 mmol) de téréphthalaldéhyde suivi de 44.69 g (soit 400 mmol) d'acide 2-mercaptopropionique à 95%. On observe une exothermie à environ 40°C et le mélange est agité à température ambiante jusqu'à dissolution du solide. On ajoute ensuite 12.22 g (soit 200 mmol) d'éthanolamine d'un seul coup ; une exothermie est observée à environ 105°C. Le milieu réactionnel est agité durant 24 heures à température ambiante sous léger débit d'azote. Après 24 heures de réaction, on prélève un aliquote de 10 g qui est dissous dans 300 ml de butanone (éthyl méthyl cétone) à 40°C. La phase organique est lavée avec 3 x 300 ml NaOH 0.6M (pH 9) et 3 x 300 ml NaCl 20% (solutions aqueuses). La phase aqueuse est réextraite avec 3 x 300 ml butanone. Les phases organiques réunies sont séchées sur Na₂SO₄, filtrées et évaporées. On obtient 7 g d'un liquide visqueux jaune qui est purifié sur gel de silice avec le mélange de solvant acétate d'éthyle et heptane. On isole un solide blanc après la purification.

¹H RMN (600 MHz, DMSO-d6) δ 7.50 - 7.27 (m, 2H), 6.00 - 5.74 (m, 1H), 4.78 (t, J = 5.5 Hz, 1H), 4.00 (qd, J = 7.1, 6.1 Hz, 1H), 3.58 (dt, J = 13.7, 5.7 Hz, 1H), 3.45 (dd, J = 11.1, 5.9 Hz, 1H), 3.35 (dd, J = 11.0, 5.6 Hz, 1H), 2.60 (dt, J = 13.6, 6.4 Hz, 1H), 1.56 - 1.41 (m, 3H).

### Monomère selon l'invention, composé (8) : Exemple 4

### Synthèse de l'acide 2-[2-[4-[5-(carboxyméthyl)-3-(2-hydroxyéthyl)-4-oxo-thiazolidin-2-yl]phényl]-3-(2-hydroxyéthyl)-4-oxo-thiazolidin-5-yl]acétique par réaction de l'acide mercaptosuccinique, d'éthanolamine et du téréphtalaldéhyde :

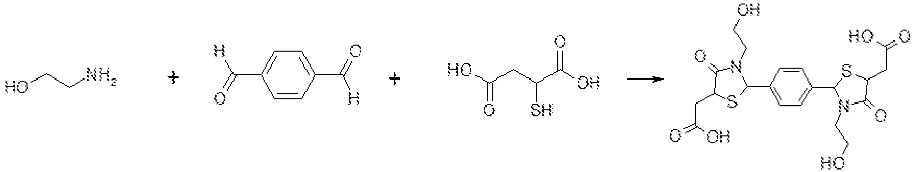

Dans un ballon à 4 cols de 100 ml préalablement séché sous vide à plus de 100°C, équipé d'un condenseur, bulleur, thermomètre et barreau magnétique, sont introduits 2.68 g (soit 20 mmol) de téréphtalaldéhyde, suivi de 60 ml de méthanol anhydre. Après dissolution sous agitation à température ambiante sont ajoutés 2.44 g (soit 40 mmol) d'éthanolamine et 6 g (soit 40 mmol) d'acide mercaptosuccinique. Le milieu réactionnel est agité à reflux pendant 48 heures sous léger débit d'azote. Le méthanol est évaporé et on obtient un liquide visqueux jaune or. Les deux monomères obtenus (spot A moins polaire que spot B) sont séparés par chromatographie sur couche mince (CCM) préparative dans l'acétate d'éthyle et méthanol (12 : 8). L'analyse par spectre FTIR ne montre pas de différence significative entre le spot A et B. L'analyse par RMN dans le DMSO-d6 montre que le spot B (le plus polaire) correspond au monomère attendu.

### Monomère selon l'invention, composé (12) : Exemple 5

### Synthèse du 3-[(4-hydroxyphényl)méthyl]-2-[4-[3-[(4-hydroxyphényl)méthyl]-5-méthyl-4-oxo-thiazolidin-2-yl]phényl]-5-méthyl-thiazolidin-4-one par réaction du téréphthaladéhyde, du 4-hydroxybenzylamine et de l'acide 2-mercaptopropionique :

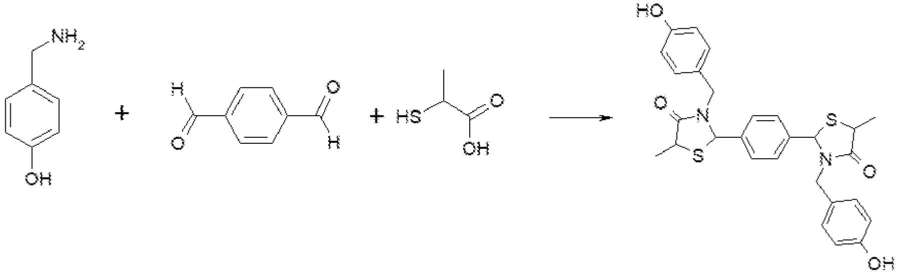

Dans un ballon à quatre cols de 250 ml préalablement séché sous vide à plus de 100°C, équipé d'un condenseur, bulleur, thermomètre et barreau magnétique, sont introduits 13.4 g (soit 100 mmol) de téréphtalaldéhyde, suivi de 60 ml de DMSO anhydre. Le mélange est agité à 50°C pour faciliter la dissolution du solide, puis laissé revenir à température ambiante. On prépare une solution de 24.63 g (soit 200 mmol) de 4-hydroxybenzylamine dans 40 ml DMSO anhydre ; afin d'aider à la solubilisation de l'amine, la solution est chauffée à 50°C. La solution aminée est ajoutée dans la solution contenant l'aldéhyde sous agitation. On ajoute pour terminer 44.69 g (soit 400 mmol) d'acide 2-mercaptopropionique à 95%. Une exothermie est constatée à environ 60°C. Le mélange réactionnel est agité à température ambiante durant 48 heures sous léger débit d'azote. Après 48 heures de réaction, on prélève une partie aliquote de 40 ml que l'on verse dans 400 g de glace ; on mélange le tout à l'aide d'une spatule et l'on obtient ainsi un mélange glace-précipité. Ce mélange est placé sur un filtre Büchner. Le mélange est rincé avec 4 portions de 250 ml d'eau glacée. Le mélange précipité-glace est déposé dans un bécher de 2L et on ajoute 500 ml d'acétone ; ce mélange est agité jusqu'à dissolution du solide. Cette solution est placée dans une ampoule à addition et est ajoutée lentement à 5 litres d'eau glacée sous agitation. Un précipité blanc se forme et est isolé par filtration Büchner. On obtient 43.2 g de solide blanc purifié par chromatographie sur gel de silice avec le mélange de solvants acétate d'éthyle et heptane (1 : 1).

¹H RMN (500 MHz, DMSO) δ 9.40 (s, 2H), 7.59 - 7.10 (m, 4H), 7.02 - 6.77 (m, 4H), 6.74 - 6.48 (m, 4H), 5.55 - 5.30 (m, 2H), 4.89 - 4.56 (m, 2H), 4.22 (d, *J* = 7.0 Hz, 1H), 4.07 (dd, *J* = 7.0, 2.7 Hz, 1H), 3.54 (dd, *J* = 14.7, 9.9 Hz, 2H), 1.75 - 1.51 (m, 3H), 1.51 - 1.32 (m, 3H).

Le composé (12) est également préparé selon le mode opératoire décrit ci-dessus à la différence près que 22.35 g d'acide 2-mercaptopropionique à 95% sont utilisés et que la réaction a lieu à 75°C. Dans ces conditions réactionnelles, il n'est pas nécessaire de purifier le produit par chromatographie sur gel de silice.

### Monomère selon l'invention, composé (3) : Exemple 6

### Synthèse de l'acide 3-[2-[4-[3-(2-carboxyéthyl)-5-méthyl-4-oxo-thiazolidin-2-yl]phényl]-5-méthyl-4-oxo-thiazolidin-3-yl]propanoique par réaction de l'acide 2-mercaptopropionique, de la β-alanine et du téréphtalaldéhyde :

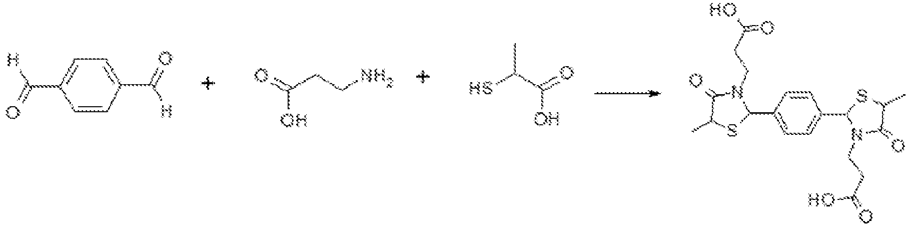

Dans un ballon à quatre cols de 250 ml préalablement séché sous vide à plus de 100°C, équipé d'un condenseur, bulleur, thermomètre et barreau magnétique, sont introduits 6.7 g de téréphtalaldéhyde (50 mmol), suivi de 30 ml de DMSO sous agitation à température ambiante. Le mélange est agité à température ambiante 5 minutes pour obtenir une solubilisation partielle de l'aldehyde, à laquelle sont ajoutés 22.35 g d'acide 2-mercaptopropionique (200 mmol) à 95% (Sigma-Aldrich, W318000) rapidement et en une seule fois. Le milieu réactionnel devient limpide avec une augmentation de la température aux environs de 42°C. Après une agitation du milieu réactionnel pendant 2 minutes à température ambiante, sont ajoutés 8.91 g de β-alanine à 99% (100 mmol) (« BioExtra », Sigma Aldrich 05160) rapidement et en une seule fois. On utilise 20 ml de DMSO au total pour rincer le matériel utilisé lors de l'introduction des composants. Le milieu réactionnel est agité à 35°C pendant 95 heures, puis refroidi à température ambiante. La moitié du mélange est traité avec 150 ml d'eau, puis acidifié avec 10 ml d'acide chlorhydrique à 10%. La phase aqueuse est extraite avec 2x100 ml d'acétate d'éthyle. Les phases organiques réunies sont séchées sur du sulfate de sodium Na₂SO₄, filtrées et évaporées à sec. Il est obtenu un liquide visqueux orange. CCM après extraction acétonitrile : MeOH : CH₃COOH (96 :4 :1). L'autre moitié du milieu réactionnel sera traité de manière identique. Pour la purification, 32 g de liquide visqueux orange sont placés dans un ballon à un col de 500 ml et sont ajoutés 50 ml d'acétate d'éthyle. Le milieu est chauffé à 50°C durant 10 minutes et 100 ml d'acétate d'éthyle sont ajoutés à la dispersion. Le mélange est chauffé à 50°C durant 30 minute et le précipité est isolé par filtration. Le solide blanc sur le filtre est rincé avec des petites portions d'acétate d'éthyle et sera séché à 50°C sous vide. La pureté et la structure du solide blanc est confirmée par analyse RMN.

¹H RMN (600 MHz, DMSO-d6) δ 12.31 (s, 2H), 7.51 - 7.22 (m, 2H), 5.83 (dt, *J* = 9.6, 2.5 Hz, 1H), 3.95 (q, *J* = 7.0 Hz, 1H), 3.61 (ddd, *J* = 8.3, 4.6, 2.8 Hz, 1H), 3.32 (s, 3H), 2.78 (ddd, *J* = 7.6, 5.1, 1.9 Hz, 1H), 2.55 (m, 2H), 2.36 - 2.16 (m, 1H), 1.45 (ddd, *J* = 38.7, 19.9, 4.0 Hz, 3H).

### Monomère selon l'invention, composé (1) : Exemple 7

### Synthèse de l'acide 3-[5-(carboxyméthyl)-4-oxo-2-phényl-thiazolidin-3-yl] propanoique par raction de l'acide mercaptosuccinique, de la β-alanine et du benzaldéhyde :

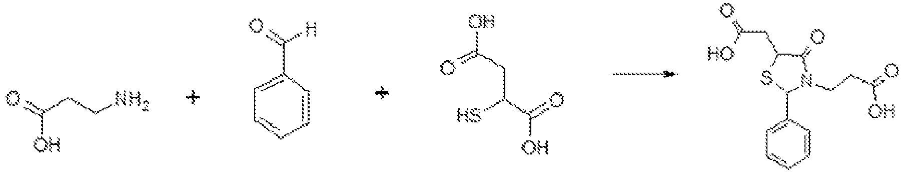

Dans un ballon à quatre cols de 100 ml préalablement séché sous vide à plus de 100°C, équipé d'un condenseur, bulleur, thermomètre et barreau magnétique, sont introduits 5.31 g (50 mmol) de benzaldéhyde (Sigma Aldrich B1334) suivi de 50 ml d'eau déminéralisée sous agitation et à température ambiante. Dans la phase troublée, sont ajoutés 4.45 g (50 mmol) de β-alanine 99% (Sigma Aldrich 05160) suivi de 7.51 g (50 mmol) d'acide thiomalique (Sigma-Aldrich, 88460). Le milieu réactionnel est chauffé à 70°C durant 71 heures avant de laisser revenir à température ambiante. Un aliquote de 25 ml du milieu réactionnel est versé dans une ampoule à brome puis est acidifiée avec 12,5 ml d'acide chlorhydrique 1M. La phase aqueuse est extraite avec 3 x 20 ml d'acétate d'éthyle. Les phases organiques réunies sont séchées avec le sulfate de sodium, filtrées et évaporées. Le solide jaune obtenu est dissout avec le minimum d'acétate d'éthyle nécessaire et on ajoute goutte à goutte du cyclohexane jusqu'à ce que la phase se trouble très légèrement. Le mélange est laissé reposer à température ambiante et après une nuit on obtient des cristaux blancs qui sont isolés par filtration. Les cristaux sont lavés avec un mélange acétate d'éthyle/cyclohexane (1 :1 volume) puis séchés à l'étuve sous vide à 50°C. La structure du produit est confirmée par analyse RMN du proton et par analyse FTIR.

¹H NMR (600 MHz, DMSO-d6) δ 12.54 (s, 2H), 7.40 - 7.37 (m, 5H), 5.77 (d, *J* = 1.9 Hz, 1H), 4.29 (ddd, *J* = 8.8, 3.6, 1.9 Hz, 1H), 3.69 (dd, *J* = 10.4, 4.7 Hz, 1H), 3.60 (ddd, *J* = 14.5, 9.0, 5.8 Hz, 1H), 2.83 - 2.65 (m, 4H).

### Monomère selon l'invention, composé (15) : Exemple 8

### Synthèse du méthyl 4-[3-[6-[2-(4-méthoxycarbonylphényl)-5-méthyl-4-oxo-thiazolidin-3-yl]hexyl]-5-méthyl-4-oxo-thiazolidin-2-yl]benzoate à partir de l'acide 2-mercaptopropionique, de l'hexaméthylènediamine and du méthyl 4-formylbenzoate :

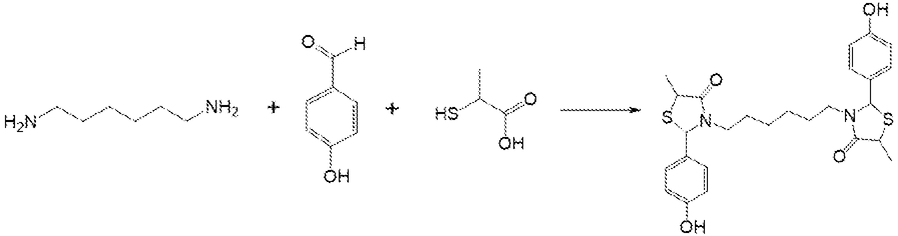

Dans un ballon à quatre cols de 500 ml préalablement séché sous vide à plus de 100°C, équipé d'un condenseur, ampoule à addition, bulleur, thermomètre et barreau magnétique, sont introduits 28.91 g (236.75 mmol) de 4-Hydroxy-benzaldéhyde (Acros Organics, 162772500), suivi de 300 ml d'éthanol sous agitation à température ambiante. Entretemps, on prépare une solution à 60°C de 13.76 g (118.37 mmol) de 1,6-Diamino-hexane à 98% (Sigma Aldrich, H11696) dans 60 ml d'éthanol. La solution est placée dans l'ampoule à addition qui est ajoutée goutte à goutte (2 gouttes/secondes) dans le ballon réactionnel sous agitation et à température ambiante. 40 ml d'éthanol sont utilisés pour le rinçage de l'ampoule à addition. Le mélange réactionnel est agité à 60°C durant 19 heures puis refroidi à température ambiante avant évaporation total du solvant. Un aliquote de 11 g (33.9 mmol) du solide jaune ainsi obtenu est placé dans un ballon à quatre cols de 250 ml préalablement séché sous vide à plus de 100°C, équipé d'un condenseur, bulleur, thermomètre et barreau magnétique. On ajoute 120 ml de N,N-dimethylacetamide à température ambiante et sous agitation, suivi de 11.36 g (101.7 mmol) d'acide 2-mercaptopropionique à 95% (Sigma-Aldrich, W318000). Le mélange réactionnel est chauffé à 116°C durant 48 heures puis refroidi à température ambiante. Le mélange est versé gentiment dans un bécher de 2L contenant 400 g de glace et en mélangeant à l'aide d'une spatule en inox. Après une heure, une masse caoutchouteuse se dépose au fond du bécher. Cette masse est placée dans un bécher de 800 ml auquel 400 ml d'acétone sont ajoutés et agité à température ambiante durant 30 minutes. Le solide est éliminé par filtration Büchner et le filtrat est évaporé à sec. Le solide blanc obtenu est séché à 50°C et sous vide. La pureté et la structure du monomère sont confirmées par analyse RMN.

¹H RMN (600 MHz, DMSO-d6) δ 9.62 (s, 2H), 7.17 (s, 4H), 6.73 (s, 4H), 5.61 (s, 2H), 3.99 (d, *J* = 78.0 Hz, 2H), 3.54 - 3.08 (m, 4H), 1.43 (d, *J* = 40.7 Hz, 6H), 1.36-1.15 (m, 4H), 1.16-0.9 (m, 4H).

### Monomère selon l'invention, composé (14) : Exemple 9

### Synthèse de l'acide 4-[3-[6-[2-(4-carboxyphényl)-5-méthyl-4-oxo-thiazolidin-3-yl]hexyl]-5-méthyl-4-oxo-thiazolidin-2-yl]benzoique :

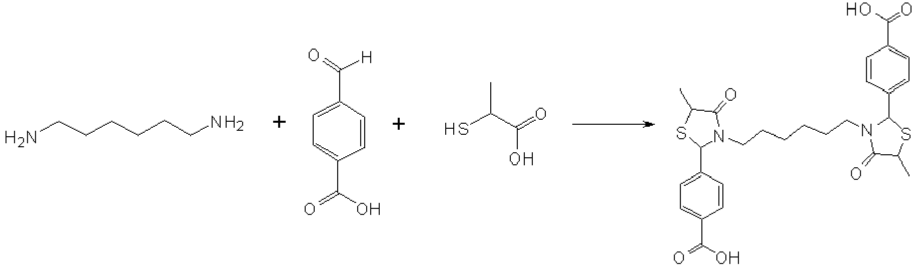

Dans un ballon à quatre cols de 250 ml préalablement séché sous vide à plus de 100°C, équipé d'un condenseur, bulleur, thermomètre et barreau magnétique, sont introduits 15.48 g (100 mmol) d'acide 4-formylbenzoïque (Sigma Aldrich 124915), suivi de 60 ml de DMSO sous agitation à température ambiante. Après dissolution de l'acide 4-formylbenzoïque, sont ajoutés 5.81 g (50 mmol) de 1,6-diamino-hexane (Sigma Aldrich, H11696) suivi de 40 ml de DMSO. Le mélange est agité à température ambiante auquel 11.17 g d'acide 2-mercaptopropionique (100 mmol) à 95% (Sigma-Aldrich, W318000) sont ajoutés rapidement et en une seule fois. Le milieu réactionnel devient orange foncé avec une augmentation de la température aux environs de 30°C. Le milieu réactionnel est agité à température ambiante durant 70 heures ; la coloration est devenue orange clair. Pour la purification, un aliquote de 40 ml du milieu réactionnel est placé dans une ampoule à brome suivi de 150 ml d'acétate d'éthyle et 250 ml d'eau déminéralisée. Après mélange, un précipité se forme dans la phase organique et la phase aqueuse est éliminée. La phase organique et le précipité sont lavés une nouvelle fois avec 250 ml d'eau déminéralisée. Après élimination de la phase aqueuse, le solide est isolé par filtration. Le précipité est agité avec 100 ml d'acétate d'éthyle à température ambiante, isolé par filtration et agité une dernière fois avec 20 ml d'éthanol. Le précipité blanc est isolé par filtration et séché à 80°C à l'étuve sous vide.

¹H RMN (600 MHz, DMSO-d6) δ 13.01 (s, 2H), 7.93 (dd, *J* = 12.4, 8.3 Hz, 4H), 7.45 (dd, *J* = 15.7, 8.3 Hz, 4H), 5.83 (d, *J* = 6.6 Hz, 2H), 4.34 - 3.83 (m, 2H), 3.72 - 3.40 (m, 4H), 1.44 (dd, *J* = 44.4, 7.0 Hz, 6H), 1.37 - 1.15 (m, 4H), 1.16 - 0.91 (m, 4H).

### Polycondensat non conforme à l'invention : Exemple 10

### Synthèse d'un polycondensat par réaction de l'acide thiolactique (acide 2-mercaptopropionique), du téréphtalaldéhyde et l'hexaméthylènediamine :

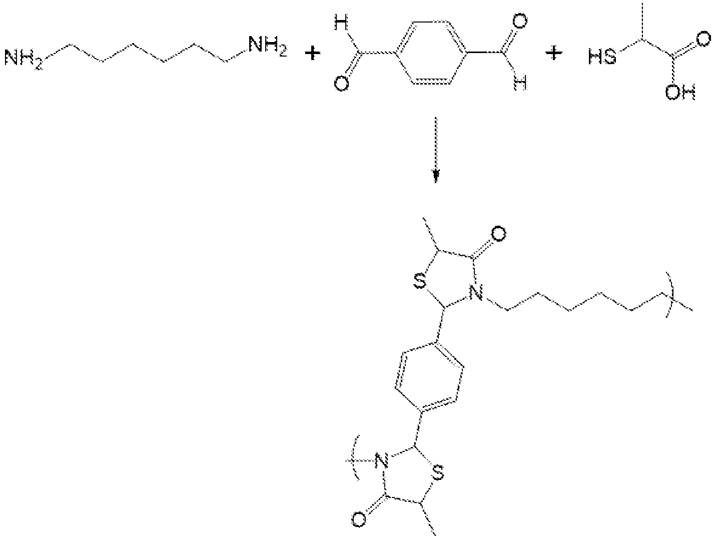

Dans un tricol de 250 ml séché et équipé d'un septum silicone, sous agitation magnétique, sous atmosphère d'azote sont ajoutés 1.3414 g de téréphtalaldéhyde (10 mmol) (Aldrich, T2207), puis 6.4 ml de diméthylsulfoxyde (DMSO). Après dissolution de l'aldéhyde sont ajoutés rapidement 1.1621 g d'hexaméthylènediamine (10 mmol) (Merck, # 8.04323.0250) dissous dans 1.6 ml DMSO (anhydre). Immédiatement après sont ajoutés 4 ml d'acide 2-mercaptopropionique à 95% (95%, Sigma Aldrich, W318000) : un précipité blanc se forme. Après une nuit sous agitation à 25°C sous courant d'azote, le milieu réactionnel est devenu transparent et jaune clair sans aucun précipité. 500 µl du milieu réactionnel est prélevé et ajouté à 1 ml d'eau : un polymère blanc précipite. Après élimination du liquide laiteux, le polymère est traité avec une solution aqueuse de soude à 1% (en masse) et rincé copieusement avec de l'eau. Le polymère blanc est séché sous vide à 70°C pendant 30 minutes, 150°C pendant 30 minutes.

L'avancement de la réaction est suivi par infra-rouge à transformée de Fourier (FTIR) : la formation du motif 4-thiazolidinone est suivie par l'apparition d'un signal à 643 cm⁻¹.

La température de transition vitreuse déterminée par analyse DSC est de 115°C. Le polymère est stable thermiquement jusqu'à 350°C. Le polymère est fragile et cassant : il est fissuré après évaporation du solvant.

### Polycondensat polyester conforme à l'invention : Exemple 11

### Synthèse d'un polycondensat polyester par réaction du monomère composé (4) avec lui même :

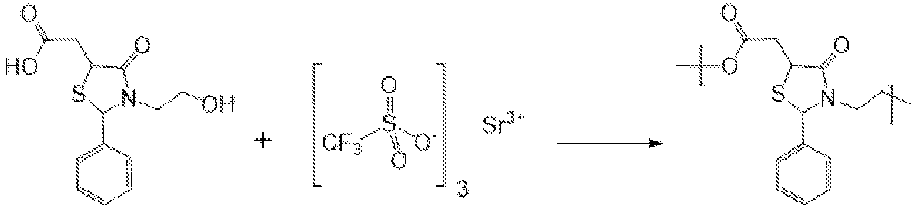

Dans un ballon à quatre cols de 25 ml préalablement séché sous vide à plus de 100°C, équipé d'un condenseur, bulleur, thermomètre et barreau magnétique, sont introduits 9.84 mg triflate de scandium Sc(III) (soit 0.02mmol), puis 563 mg THZP-2003A dissous dans 3ml d'acétonitrile anhydre. Le mélange est agité à 75°C sous azote pendant 24h. Le mélange réactionnel est ensuite chauffé à 85 °C pour distiller le solvent et la température de milieu réactionnel est augmentée jusqu'à 185 °C (10 °C tous les 10 minutes) pour compléter la réaction de polymérisation. La polymérisation est suivie par FTIR : diminution du signal à 3600 cm⁻¹ (fonction OH) avec le degré de polymérisation du monomère et changement d'absorbance du groupe carbonyle de 1500 cm⁻¹ (fonction COOH) à 1750 cm⁻¹ (fonction ester).

### Polycondensat polyamide conforme à l'invention : Exemple 12

### Synthèse d'un polycondensat polyamide par réaction du monomère composé (3) avec la 1,6-hexaméthylènediamine :

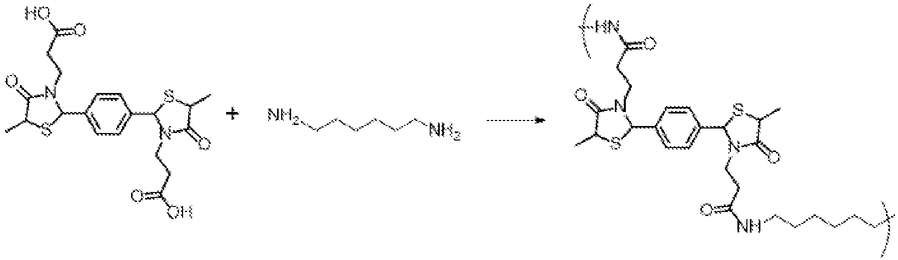

La 1,6-hexaméthylènediamine (HDMA, Sigma Aldrich H11696) (344.6mg) dissolute dans 1.5 ml d'éthanol anhydre est dégazée sous azote à 60°C. Ensuite, 134.1 mg de composé (3) dissous dans 3.5 ml d'éthanol sont mélangés à 150 µl de HDMA à 60°C. La solution résultante est refroidie dans un bain de glace pour faire précipiter le sel. Les cristaux obtenus sont séchés à 60°C sous vide et sont chauffés dans une capsule placé dans un appareil de calorimétrie à balayage différentiel (DSC) sous azote de -80°C à 400°C selon une rampe de température de 10°C/min.

Par analyse DSC sur une plage de température allant de -80°C à 400°C, il est mesuré au second cycle une température de transition vitreuse de 107°C et une première fusion s'étalant de 220°C à environ 330°C avec un pic à 288°C et une deuxième fusion avec un pic à 353°C.

### Polycondensat polyuréthane conforme à l'invention : Exemple 13

### Synthèse d'un polycondensat polyuréthane par réaction du monomère composé (5) avec le diisocyanate prépolymère « Adiprene LFP 950A » (prépolymère PPDI), (Chemtura, commercialisé par Lanxess, USA) :

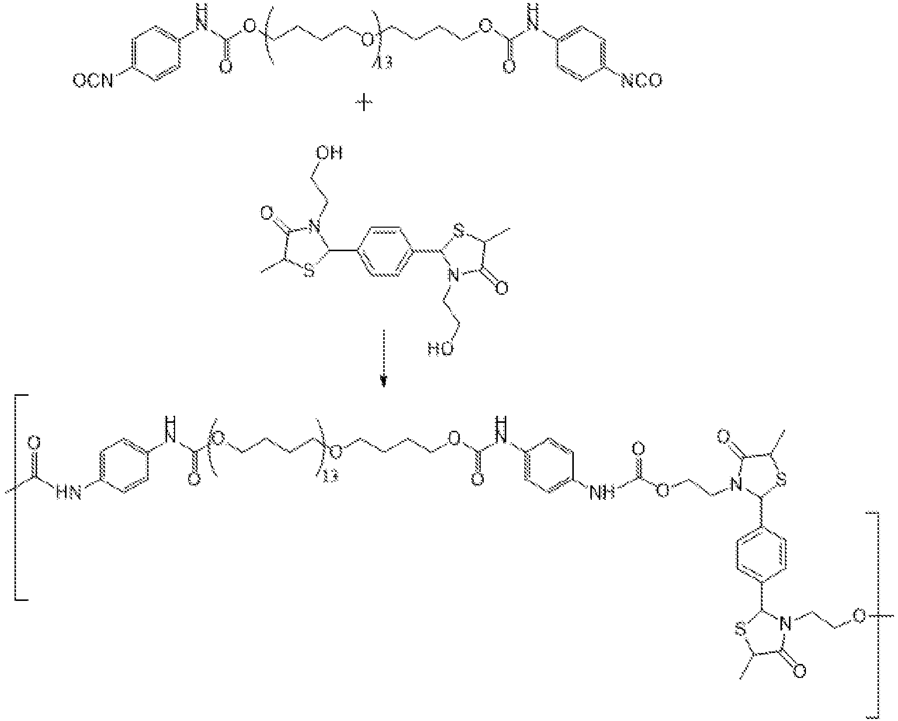

Le prépolymère « Adiprene LFP 950A » (4.16g) (Chemtura, Italy) est chauffé 20 minutes à 80°C. Une fois fondu, il est dégazé sous vide (10 mbar). On ajoute le composé (5) (0.856g) et le mélange réactionnel est bien mélangé à l'aide d'une spatule. Le tout est placé à l'étuve à 110°C et dégazé une nouvelle fois sous vide pendant 10 minutes. Le produit obtenu est versé dans un moule préalablement enduit d'un démoulant Gorapur LS1040-33). Le moule est recouvert d'un poids de 3.5 kg (préchauffé) et placé à l'étuve à 150°C durant 4 heures. A la fin des 4 heures, il ne reste plus de isocyanate libre selon analyse FTIR à 2264.5 cm⁻¹.

Par analyse DSC sur une plage de température allant de -130°C à 250°C, il est mesuré une température de transition vitreuse de -56°C au troisième cycle et il n'y a pas de fusion sur la plage de température balayée tant au premier cycle qu'aux deuxième et troisième cycles. L'absence de fusion dans cette gamme de température assure un maintien des modules du polyuréthane et donc de ses propriétés mécaniques.

Par analyse DMA sur une plage de température allant de -120°C à 180°C, il est mesuré une température de transition vitreuse (Tg) de -58°C, un module de Young (E') à 25°C de 42.7 MPa et à 50°C de 34.1 MPa et une élongation à la rupture de 600%. L'éprouvette a pour dimension H = 10, E = 3.66, L = 4.4.

Par ailleurs, il n'est pas observé de déformation rémanente pour des élongations inférieures à 10%, ce qui traduit que le polyuréthane est élastique à des déformations inférieures à 10% et n'est donc pas sujet au fluage sous l'effet d'une contrainte en traction.

### Polycondensat polyuréthane conforme à l'invention : Exemple 14

Synthèse d'un polycondensat polyuréthane par réaction du monomère composé (5) avec le diisocyanate prépolymère « Adiprene LFM C525», polycaprolactone α,ω-diphénylméthane diisocyanate » (Chemtura, Italy) (prépolymère MDI) :
Le prépolymère « Adiprene LFM C525» (100 parts, en masse) est chauffé 20 minutes à 130°C. Une fois fondu, il est dégazé sous vide (10 mbar). On ajoute le 1,4-butanediol (5.9 parts) et le mélange réactionnel est bien homogénéisé avec un mélangeur (« State Mix »). Le tout est coulé dans un moule préalablement chauffé à 120°C et cuit pendant 16 heures à 120°C.

Par analyse DSC sur une plage de température allant de -80°C à 260°C sous azote, il est mesuré une température de transition vitreuse de -7°C et il n'y a pas de fusion sur la plage de température balayée. L'absence de fusion dans cette gamme de température assure un maintien des modules du polyuréthane et donc de ses propriétés mécaniques.

Par ailleurs, il n'est pas observé de déformation rémanente pour des élongations inférieures à 10%, ce qui traduit que le polyuréthane est élastique à des déformations inférieures à 10% et n'est donc pas sujet au fluage sous l'effet d'une contrainte en traction.

### Polycondensat polyuréthane non conforme à l'invention : Exemple 15

Synthèse d'un polycondensat polyuréthane par réaction du 1,4-butanediol, monomère non conforme, avec le diisocyanate prépolymère « Adiprene LFM C525», polycaprolactone α,ω-diphénylméthane diisocyanate :
Le prépolymère « Adiprene LFM C525» (100 parts, en masse) est chauffé 20 minutes à 130°C. Une fois fondu, il est dégazé sous vide (10 mbar). On ajoute le 1,4-butanediol (5.9 parts) et le mélange réactionnel est bien homogénéisé avec un mélangeur (« State Mix »). Le tout est coulé dans un moule préalablement chauffé à 120°C et cuit pendant 16 heures à 120°C.

L'analyse DSC réalisée sur une plage de température allant de -80°C à 250°C sous azote montre que le polyuréthane commence à fondre à partir de la température ambiante : ses modules E' et E" chutent, ce qui traduit que le polyuréthane perd alors toutes ses propriétés mécaniques à partir de la température ambiante.

### Polycondensat polyuréthane non conforme à l'invention : Exemple 16

Synthèse d'un polycondensat polyuréthane par réaction de l'hydroquinone bis(2-hydroxyéthyléther) (HQEE) (Vibracure 2101), monomère non conforme, avec le diisocyanate prépolymère « Adiprene LFM C525» :
Le prépolymère « Adiprene LFM C525» (100 parts) (Chemtura, Italy) est chauffé 20 minutes à 130°C. Une fois fondu, il est dégazé sous vide (10 mbar). On ajoute le diol HQEE (11.8 parts) et le mélange réactionnel est bien homogénéisé avec un mélangeur (« State Mix »). Le tout est coulé dans un moule préalablement chauffé à 120°C et cuit pendant 16 heures à 120°C.

Par analyse DSC sur une plage de température allant de -80°C à 250°C sous azote, il est mesuré une température de transition vitreuse de -32°C et une fusion s'étalant d'environ 92°C à environ 180°C avec deux pics de fusion à 152°C et à 172°C.

Le premier cycle de la DSC montre que le polyuréthane est thermoplastique, puisqu'en plus d'une Tg vers -40°C, il présente deux autres Tg, respectivement vers 45°C et 89°C, et qu'il commence à fondre à partir d'environ 140°C.

Par analyse DMA sur une plage de température allant de -120°C à 180°C, il est mesuré une température de transition vitreuse (Tg) de -27°C et un module de Young à 50°C de 34 MPa.. L'éprouvette a pour dimension H = 10, E = 3.02, L = 4.9.

Par ailleurs, il est observé une déformation rémanente pour des élongations inférieures à 10%, ce qui traduit que le polyuréthane n'est pas élastique à des déformations inférieures à 10% et est donc sujet au fluage sous l'effet d'une contrainte en traction.

### Polycondensat polyuréthane conforme à l'invention : Exemple 17

Synthèse d'un polycondensat polyuréthane par réaction du monomère composé (5) avec le diisocyanate prépolymère « Adiprene LFP E560 », (Chemtura, commercialisé par Lanxess, USA) (prépolymère PPDI) :
Le prépolymère « Adiprene LFP E560 » (100.0g) (Chemtura, Italy) est chauffé 20 minutes à 100°C. Une fois fondu, il est dégazé sous vide (<1 mbar) jusqu'à ce qu'il ne se forme plus de bulles dans le prépolymère (pendant moins de 3min). On ajoute le composé (5) en poudre (20.76g), séché 16h à 100 °C, et le mélange réactionnel est bien mélangé à l'aide d'un mélangeur (« State Mix »), puis est dégazé à nouveau. Le produit obtenu est versé dans un moule préalablement enduit d'un démoulant « BYK A-501 » commercialisé par la société BYK-Chemie, Allemagne. Le moule est recouvert d'un poids de 3.5 kg (préchauffé) et placé à l'étuve à 150°C durant 4 heures. A la fin des 4 heures, il ne reste plus d'isocyanate libre selon l'analyse FTIR à 2264.5 cm⁻¹.

Par analyse DSC sur une plage de température allant de -130°C à 250°C, il est mesuré une température de transition vitreuse de -48.8°C au deuxième cycle et il n'y a pas de fusion sur la plage de température balayée au deuxième cycle. L'absence de fusion dans cette gamme de température assure un maintien des modules du polyuréthane et donc de ses propriétés mécaniques.

Par analyse DMA sur une plage de température allant de -120°C à 180°C, il est mesuré une température de transition vitreuse (Tg) de -40°C, un module de Young (E') à 25°C de 75.7 MPa et à 20°C et 59.2 MPa à 100 °C.

Les résultats montrent que les monomères selon l'invention donnent accès à une large variété de polycondensats, polyamide, polyester et polyuréthane, et sans avoir recours au cours de la réaction de polycondensation à un thiol odorant, comme l'acide 2-mercaptopropionique.

Les résultats montrent aussi que les polycondensats selon l'invention sont bien moins fragiles que le polycondensat obtenu par polycondensation de l'acide 2-mercaptopropionique, de l'hexaméthylènediamine et du téréphtalaldéhyde et décrit dans le journal Polymer Chemistry, 2014, 5, 2695.

Les résultats montrent que les polyuréthanes contenant des motifs 4-thiazolidinone dans leurs unités monomères présentent des propriétés supérieures à celles des polyuréthanes sans motif 4-thiazolidinone au sein de leur chaîne.

En effet, l'absence de fusion des polyuréthanes selon l'invention à des températures aussi hautes que 250°C assure un maintien de leurs propriétés mécaniques à ces températures, ce qui n'est pas le cas des polyuréthanes sans motif 4-thiazolidinone au sein de leur chaîne.

Par ailleurs, les polyuréthanes selon l'invention ont l'avantage sur leurs homologues non conformes d'avoir un comportement élastique à des déformations inférieurs à 10%, ce qui assure aussi une stabilité dimensionnelle de toute pièce faite en polyuréthane selon l'invention et soumise à des déformations inférieures à 10%.

L'amélioration des propriétés est attribuée à la présence des motifs 4-thiazolidinone au sein de la chaîne polymère des polycondensats polyamide, polyester et polyuréthane.

## Revendications

1. Monomère qui contient un motif 4-thiazolidinone relié à un noyau benzénique et qui contient deux fonctions choisies parmi hydroxyle et carboxyle, lequel monomère est de formule (I-a) ou (I-b) dans lesquelles Y est hydroxyle ou carboxyle, Q est alkyle ou carboxyalkyle, R₂ est hydrogène, hydroxyle, carboxyle, alkyle ou hydroxyalkyle, Y' est hydroxyle ou carboxyle ; Q' est alkyle ou carboxyalkyle, 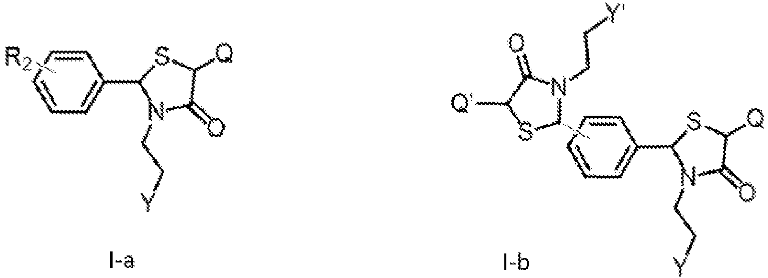 ou de formule (I-c) dans laquelle Y est hydroxyle ou carboxyle, Q est alkyle ou carboxyalkyle, Y' est hydroxyle ou carboxyle ; Q' est alkyle ou carboxyalkyle, x est un entier allant de 0 à 2. 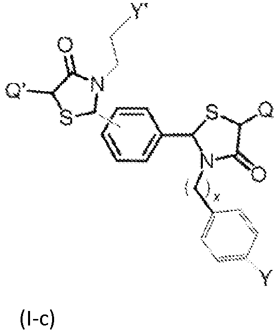 ou bien de formule (II-1) dans laquelle Q est alkyle ou carboxyalkyle, R₃ est hydrogène, hydroxyle, carboxyle, alkyle ou hydroxyalkyle, R₁ est alkyle et A₁, groupe carboné divalent qui contient des atomes de carbone, est un alcanediyle ou un alcanediyle interrompu par un ou plusieurs atomes d'oxygène, m est un entier allant de 0 à 4.

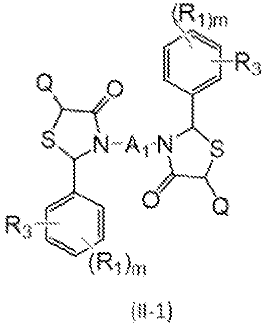

2. Monomère selon la revendication 1, dans lequel m est égal à 0.

3. Monomère selon l'une quelconque des revendications 1 à 2 dans lequel les alkyles représentés par Q et Q' sont méthyle, les carboxyalkyles représentés par Q et Q' sont carboxyméthyle, les hydroxyalkyles représenté par R₂ et R₃ sont hydroxyméthyle.

4. Monomère selon l'une quelconque des revendications 1 à 3, dans lequel R₂ est hydrogène, hydroxyle, carboxyle ou hydroxyméthyle.

5. Procédé de synthèse d'un monomère défini à l'une quelconque des revendications 1 à 4 qui comprend la réaction d'un aldéhyde de formule (II), d'un composé (III) et d'un composé de formule (IV) 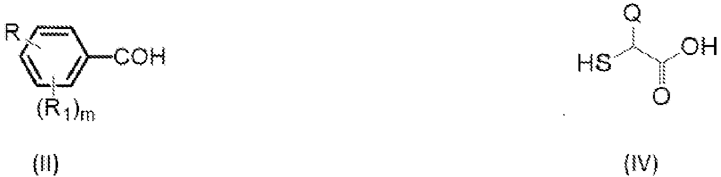
le composé (III) étant de formule Y-A-NH₂ ou de formule H₂N-A₁-NH₂,
dans lesquelles Y est hydroxyle ou carboxyle, Q est alkyle ou carboxyalkyle, A est un éthanediyle, A₁ est un alcanediyle ou un alcanediyle interrompu par un ou plusieurs atomes d'oxygène, R₁ est alkyle, m est un entier allant de 0 à 4, R est hydrogène, hydroxyle, carboxyle, alkyle, hydroxyalkyle ou une fonction aldéhyde.

6. Procédé de synthèse d'un polyamide qui comprend la réaction d'un monomère défini à l'une quelconque des revendications 1 à 4 avec un deuxième monomère contenant deux fonctions choisies parmi amine et isocyanate, le monomère défini à l'une quelconque des revendications 1 à 4 contenant deux fonctions carboxyles.

7. Procédé de synthèse d'un polyester qui comprend la réaction d'un monomère défini à l'une quelconque des revendications 1 à 4 avec un deuxième monomère, les deux monomères contenant chacun une fonction carboxyle et une fonction hydroxyle ou bien l'un des deux monomères contenant deux fonctions hydroxyles et l'autre deux fonctions carboxyles.

8. Procédé de synthèse d'un polyuréthane qui comprend la réaction d'un monomère de défini à l'une quelconque des revendications 1 à 4 avec un deuxième monomère contenant deux fonctions isocyanates, le monomère défini à l'une quelconque des revendications 1 à 4 contenant deux fonctions hydroxyles.

9. Procédé selon l'une quelconque des revendications 6 à 8 dans lequel le deuxième monomère est remplacé en partie ou en totalité par un prépolymère qui contient deux fonctions réactives identiques à celle du deuxième monomère.

10. Polycondensat susceptible d'être obtenu par un procédé défini à l'une quelconque des revendications 6 à 9, le polycondensat étant un polyamide, un polyester ou un polyuréthane et contenant des unités monomères comportant des motifs 4-thiazolidinone.

## Patentansprüche

1. Monomer, das eine 4-Thiazolidinon-Einheit enthält, die mit einem Benzolring verbunden ist und zwei Funktionen enthält, die aus Hydroxyl und Carboxyl ausgewählt sind, wobei das Monomer der Formel (I-a) oder (I-b), in denen Y für Hydroxyl oder Carboxyl steht, Q für Alkyl oder Carboxyalkyl steht, R₂ für Wasserstoff, Hydroxyl, Carboxyl, Alkyl oder Hydroxyalkyl steht, Y' für Hydroxyl oder Carboxyl steht; Q' für Alkyl oder Carboxyalkyl steht, 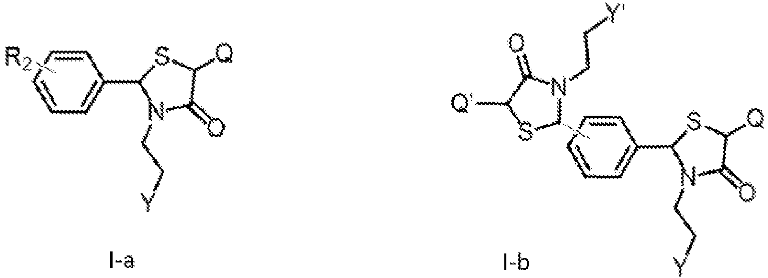 oder der Formel (I-c), in der Y für Hydroxyl oder Carboxyl steht, Q für Alkyl oder Carboxyalkyl steht, Y' für Hydroxyl oder Carboxyl steht; Q' für Alkyl oder Carboxyalkyl steht, x für eine ganze Zahl von 0 bis 2 steht, 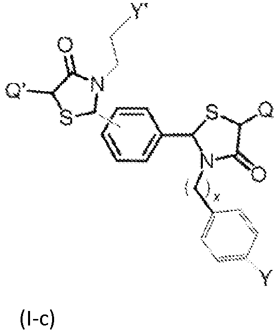 oder der Formel (II-1), in der Q für Alkyl oder Carboxyalkyl steht, R₃ für Wasserstoff, Hydroxyl, Carboxyl, Alkyl oder Hydroxyalkyl steht, R₁ für Alkyl steht und A₁, eine zweiwertige Kohlenstoffgruppe, die Kohlenstoffatome enthält, für ein Alkandiyl oder ein Alkandiyl, das durch ein oder mehrere Sauerstoffatome unterbrochen ist, steht, m für eine ganze Zahl von 0 bis 4 steht, entspricht.

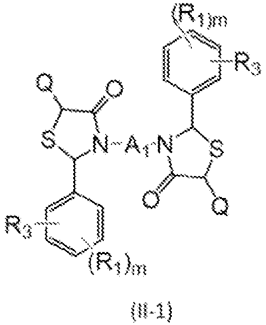

2. Monomer nach Anspruch 1, wobei m gleich 0 ist.

3. Monomer nach einem der Ansprüche 1 bis 2, wobei es sich bei den durch Q und Q' wiedergegebenen Alkylgruppen um Methyl handelt, es sich bei den durch Q und Q' wiedergegebenen Carboxyalkylgruppen um Carboxymethyl handelt, es sich bei den durch R₂ und R₃ wiedergegebenen Hydroxyalkylgruppen um Hydroxymethyl handelt.

4. Monomer nach einem der Ansprüche 1 bis 3, wobei R₂ für Wasserstoff, Hydroxyl, Carboxyl oder Hydroxymethyl steht.

5. Verfahren zur Synthese eines Monomers gemäß einem der Ansprüche 1 bis 4, bei dem man einen Aldehyd der Formel (II), eine Verbindung (III) und eine Verbindung der Formel (IV) umsetzt 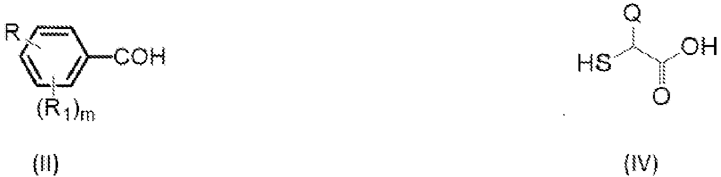
wobei die Verbindung (III) die Formel Y-A-NH₂ oder die Formel H₂N-A₁-NH₂ aufweist,
wobei Y für Hydroxyl oder Carboxyl steht, Q für Alkyl oder Carboxyalkyl steht, A für ein Ethandiyl steht, A₁ für ein Alkandiyl oder ein Alkandiyl, das durch ein oder mehrere Sauerstoffatome unterbrochen ist, steht, R₁ für Alkyl steht, m für eine ganze Zahl von 0 bis 4 steht, R für Wasserstoff, Hydroxyl, Carboxyl, Alkyl, Hydroxyalkyl oder eine Aldehydfunktion stellt.

6. Verfahren zur Synthese eines Polyamids, bei dem man ein Monomer gemäß einem der Ansprüche 1 bis 4 mit einem zweiten Monomer mit zwei Funktionen, die aus Amin und Isocyanat ausgewählt sind, umsetzt, wobei das Monomer gemäß einem der Ansprüche 1 bis 4 zwei Carboxylfunktionen enthält.

7. Verfahren zur Synthese eines Polyesters, bei dem man ein Monomer gemäß einem der Ansprüche 1 bis 4 mit einem zweiten Monomer umsetzt, wobei die beiden Monomere jeweils eine Carboxylfunktion und eine Hydroxylfunktion enthalten oder eines der beiden Monomere zwei Hydroxylfunktionen enthält und das andere zwei Carboxylfunktionen enthält.

8. Verfahren zur Synthese eines Polyurethans, bei dem man ein Monomer gemäß einem der Ansprüche 1 bis 4 mit einem zweiten Monomer mit zwei Isocyanatfunktionen umsetzt, wobei das Monomer gemäß einem der Ansprüche 1 bis 4 zwei Hydroxylfunktionen enthält.

9. Verfahren nach einem der Ansprüche 6 bis 8, bei dem das zweite Monomer teilweise oder ganz durch ein Prepolymer ersetzt wird, das zwei reaktive Funktionen enthält, die mit derjenigen des zweiten Monomers identisch sind.

10. Polykondensat, erhältlich durch ein Verfahren gemäß einem der Ansprüche 6 bis 9, wobei das Polykondensat ein Polyamid, ein Polyester oder ein Polyurethan ist und Monomereinheiten enthält, die 4-Thiazolidinon-Einheiten umfassen.

## Claims

1. Monomer which contains a 4-thiazolidinone unit connected to a benzene nucleus and which contains two functions selected from hydroxyl and carboxyl, which monomer is of formula (I-a) or (I-b) in which Y is hydroxyl or carboxyl, Q is alkyl or carboxyalkyl, R₂ is hydrogen, hydroxyl, carboxyl, alkyl or hydroxyalkyl, Y' is hydroxyl or carboxyl; Q' is alkyl or carboxyalkyl, 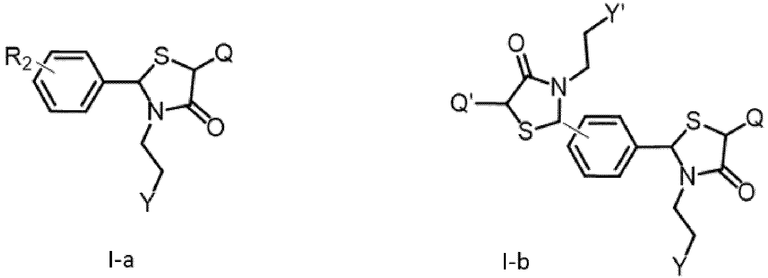 or of formula (I-c) in which Y is hydroxyl or carboxyl, Q is alkyl or carboxyalkyl, Y' is hydroxyl or carboxyl; Q' is alkyl or carboxyalkyl, x is an integer ranging from 0 to 2, 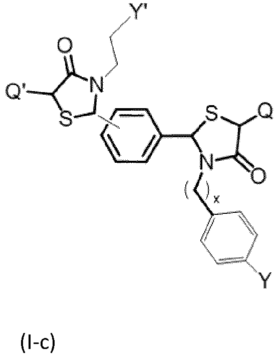 or else of formula (II-1) in which Q is alkyl or carboxyalkyl, R₃ is hydrogen, hydroxyl, carboxyl, alkyl or hydroxyalkyl, R₁ is alkyl and A₁, a divalent carbon group which contains carbon atoms, is an alkanediyl or an alkanediyl interrupted by one or more oxygen atoms, m is an integer ranging from 0 to 4.

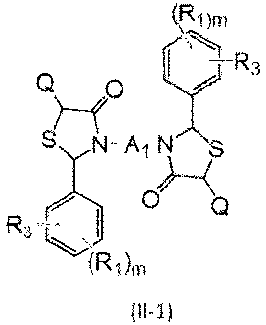

2. Monomer according to Claim 1, wherein m is equal to 0.

3. Monomer according to either one of Claims 1 and 2, wherein the alkyls represented by Q and Q' are methyl, the carboxyalkyls represented by Q and Q' are carboxymethyl, the hydroxyalkyls represented by R₂ and R₃ are hydroxymethyl.

4. Monomer according to any one of Claims 1 to 3, wherein R₂ is hydrogen, hydroxyl, carboxyl or hydroxymethyl.

5. Process for synthesizing a monomer defined in any one of Claims 1 to 4 which comprises the reaction of an aldehyde of formula (II), a compound (III) and a compound of formula (IV) 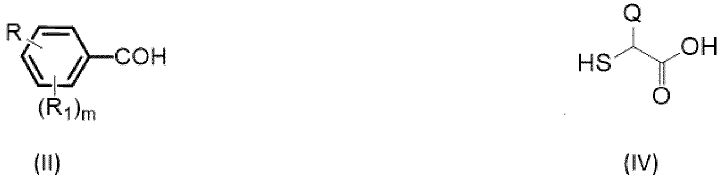
the compound (III) being of formula Y-A-NH₂ or of formula H₂N-A₁-NH₂,
in which Y is hydroxyl or carboxyl, Q is alkyl or carboxyalkyl, A is an ethanediyl, A₁ is an alkanediyl or an alkanediyl interrupted by one or more oxygen atoms, R₁ is alkyl, m is an integer ranging from 0 to 4, R is hydrogen, hydroxyl, carboxyl, alkyl, hydroxyalkyl or an aldehyde function.

6. Process for synthesizing a polyamide which comprises the reaction of a monomer defined in any one of Claims 1 to 4 with a second monomer containing two functions selected from amine and isocyanate, the monomer defined in any one of Claims 1 to 4 containing two carboxyl functions.

7. Process for synthesizing a polyester which comprises the reaction of a monomer defined in any one of Claims 1 to 4 with a second monomer, both monomers each containing a carboxyl function and a hydroxyl function or alternatively one of the two monomers containing two hydroxyl functions and the other containing two carboxyl functions.

8. Process for synthesizing a polyurethane which comprises the reaction of a monomer defined in any one of Claims 1 to 4 with a second monomer containing two isocyanate functions, the monomer defined in any one of Claims 1 to 4 containing two hydroxyl functions.

9. Process according to any one of Claims 6 to 8, wherein the second monomer is partly or totally replaced by a prepolymer which contains two reactive functions identical to that of the second monomer.

10. Polycondensate capable of being obtained by a process defined in any one of Claims 6 to 9, the polycondensate being a polyamide, a polyester or a polyurethane and containing monomer units comprising 4-thiazolidinone units.
